# EUROPEAN PATENT SPECIFICATION

(11) **EP 3 744 260 B1**
(45) Date of publication and mention of the grant of the patent: **15.11.2023**
(21) Application number: 20177184.7
(22) Date of filing: 28.05.2020
(51) Int. Cl.: A61B 17/072

(54) **NOZZLE FLUID INGRESS PREVENTION FEATURES FOR SURGICAL STAPLER**
FUNKTIONEN ZUR VERHINDERUNG DES EINDRINGENS VON DÜSENFLÜSSIGKEIT FÜR EIN CHIRURGISCHES KLAMMERGERÄT
FONCTIONS DE PRÉVENTION CONTRE LA PÉNÉTRATION DE FLUIDE DE BUSE POUR AGRAFEUSE CHIRURGICALE

(30) Priority: 28.05.2019 US 201916423841
(43) Date of publication of application: 02.12.2020
(73) Proprietor: Ethicon LLC, 00969 Guaynabo (PR)
(72) Inventor: CORSETTO, Laura R., West Orange, NJ 07052 (US); FANELLI, Nicholas, Cincinnati, OH 45242 (US); BABER, Daniel L., Cincinnati, OH 45242 (US); ARONHALT, Taylor W., Cincinnati, OH 45242 (US); RECTOR, Jason M., Cincinnati, OH 45242 (US); OGZEWALLA, James Bernar, Dayton, OH 45342 (US)
(74) Representative: Carpmaels & Ransford LLP

(56) References cited:
- US-A- 4 936 842
- US-A- 5 467 911
- US-A- 5 820 009
- US-A- 6 099 537
- US-A1- 2007 084 898
- US-A1- 2019 000 465

## Description

### BACKGROUND

Endoscopic surgical instruments may be preferred over traditional open surgical devices in certain instances to create a smaller surgical incision in the patient and thereby reduce the post-operative recovery time and complications. Examples of endoscopic surgical instruments include surgical staplers. Some such staplers are operable to clamp down on layers of tissue, cut through the clamped layers of tissue, and drive staples through the layers of tissue to substantially seal the severed layers of tissue together near the severed ends of the tissue layers. Merely exemplary surgical staplers are disclosed in U.S. Pat. No. 7,404,508, entitled "Surgical Stapling and Cutting Device," issued July 29, 2008; U.S. Pat. No. 7,721,930, entitled "Disposable Cartridge with Adhesive for Use with a Stapling Device," issued May 25, 2010; U.S. Pat. No. 8,408,439, entitled "Surgical Stapling Instrument with An Articulatable End Effector," issued April 2, 2013; U.S. Pat. No. 8,453,914, entitled "Motor-Driven Surgical Cutting Instrument with Electric Actuator Directional Control Assembly," issued June 4, 2013; U.S. Pat. No. 9,186,142, entitled "Surgical Instrument End Effector Articulation Drive with Pinion and Opposing Racks," issued November 17, 2015; and U.S. Pat. No. 9,795,379, entitled "Surgical Instrument with Multi-Diameter Shaft," issued October 24, 2017. The disclosure of each of the above-cited U.S. patents is referred to herein.

While the surgical staplers referred to above are described as being used in endoscopic procedures, such surgical staplers may also be used in open procedures and/or other non-endoscopic procedures. By way of example only, a surgical stapler may be inserted through a thoracotomy, and thereby between a patient's ribs, to reach one or more organs in a thoracic surgical procedure that does not use a trocar as a conduit for the stapler. Of course, surgical staplers may be used in various other settings and procedures.

While several surgical instruments and systems have been made and used, it is believed that no one prior to the inventors has made or used the invention described in the appended claims.

Document US 6 099 537 A discloses a medical treatment instrument with a treatment section for treating issue, an insertion section which has at one end the treatment section, and an operating section at the other end of the insertion section and operates the treatment section, and has a fixed portion and a movable portion. The instrument includes sealing members to seal the closure of the instrument.

Document US 4 936 842 A discloses a disposable bipolar electrosurgical probe which can be readily applied to the tissue to be treated, then discarded. The probe comprises an elongated substrate member having a peripheral surface of dielectric material and a longitudinal axis extending between a working end and an attachment end. At least a pair of electrically isolated electrodes extend continuously on the peripheral surface of the substrate member between an active region at the working end and an electrical terminal region at the attachment end for quick connection and/or disconnection, respectively, to opposite poles of an RF generator. A handle with a quick disconnect mechanism is provided to physically support the probe and enable it to be selectively energized. The device includes an o-ring seal to seal the probe housing.

### BRIEF DESCRIPTION OF THE DRAWINGS

The accompanying drawings, which are incorporated in and constitute a part of this specification, illustrate embodiments of the invention, and, together with the general description of the invention given above, and the detailed description of the embodiments given below, serve to explain the principles of the present invention.
FIG. 1 depicts a perspective view of an exemplary surgical instrument having a handle assembly and an interchangeable shaft assembly;
FIG. 2 depicts a partially exploded perspective view of the surgical instrument of FIG. 1, showing the interchangeable shaft assembly separated from the handle assembly, where the shaft assembly includes a nozzle
FIG. 3A depicts a side elevational view of the surgical instrument of FIG. 1, with a body of the handle assembly omitted, showing a closure trigger of the handle assembly in an unactuated position;
FIG. 3B depicts a side elevational view of the surgical instrument of FIG. 1, with a body of the handle assembly omitted, showing a closure trigger of the handle assembly in an actuated position;
FIG. 4 depicts another perspective view of the surgical instrument of FIG. 1 in a separated state, showing additional details of a distal end of the handle assembly and a mating proximal end of the interchangeable shaft assembly;
FIG. 5 depicts an exploded perspective view of the nozzle of FIG. 1, where the nozzle includes an upper distal nozzle housing, a lower distal nozzle housing, and a proximal nozzle housing;
FIG. 6 depicts a perspective view of a first exemplary alternative shaft assembly that includes a first exemplary alternative nozzle and a first exemplary fluid blocker disposed between upper and lower distal nozzle housings of the nozzle;
FIG. 7 depicts an enlarged bottom view of the upper distal nozzle housing of FIG. 6;
FIG. 8 depicts an enlarged top view of the fluid blocker and the lower distal nozzle housing of FIG. 6, where the fluid blocker is disposed within a recess of the lower distal nozzle housing;
FIG. 9 depicts a perspective view of the fluid blocker of FIG. 6; This fluid blocker does not fall under the claimed invention;
FIG. 10 depicts a top view of a second exemplary fluid blocker disposed within a recess of the lower distal nozzle housing of FIG. 6;
FIG. 11 depicts a perspective view of the first and second portions of the fluid blocker of FIG. 10; This fluid blocker falls under the claimed invention;
FIG. 12 depicts a second exemplary alternative shaft assembly that includes a second exemplary alternative nozzle and a third exemplary fluid blocker;
FIG. 13 depicts a partial cross-sectional perspective view of the shaft assembly of FIG. 12, where the fluid blocker is shown in cross-section to expose upper and lower distal nozzle housings;
FIG. 13A depicts a cross-sectional view of the shaft assembly of FIG. 12 taken along line 13A-13A of FIG. 13;
FIG. 14 depicts a perspective view of a third exemplary alternative shaft assembly that includes a third exemplary alternative nozzle and a fourth exemplary fluid blocker between upper and lower distal nozzle housings of the nozzle, where the upper distal nozzle housing is shown as transparent;
FIG. 15 depicts a perspective view of the lower distal nozzle housing and the fluid blocker of FIG. 14;
FIG. 16 depicts a perspective view of a fourth exemplary alternative nozzle and a fifth exemplary fluid blocker, where the fluid blocker is disposed between a proximal nozzle housing and upper and lower distal nozzle housings of the nozzle;
FIG. 17 depicts a perspective view of the fluid blocker and the proximal nozzle housing of FIG. 16, where the fluid blocker is coupled with the proximal nozzle housing;
FIG. 18 depicts a perspective view of a fourth exemplary alternative shaft assembly that includes a fifth exemplary alternative nozzle and a sixth exemplary fluid blocker covering at least a portion of the nozzle;
FIG. 19 depicts a perspective view of the nozzle of FIG. 18, where the fluid blocker is shown in cross-section;
FIG. 19A depicts a cross-sectional view of the nozzle of FIG. 19 taken along line 19A-19A of FIG. 19;
FIG. 20 depicts a perspective view of a sixth exemplary alternative nozzle and a seventh exemplary fluid blocker disposed between upper and lower distal nozzle housings of the nozzle;
FIG. 20A depicts a perspective view of an enlarged portion of the fluid blocker and the upper and lower distal nozzle housings of FIG. 20;
FIG. 21 depicts a perspective view of a seventh exemplary alternative nozzle and an eighth exemplary fluid blocker, where the fluid blocker is disposed between upper and lower distal nozzle housings of the nozzle;
FIG. 22A depicts a schematic cross-sectional view of an enlarged portion of the fluid blocker and the upper and lower distal nozzle housings of FIG. 21, prior to the fluid blocker engaging the lower distal nozzle housing;
FIG. 22B depicts a schematic cross-sectional view of the enlarged portion of the fluid blocker and the upper and lower distal nozzle housings of FIG. 22A, but after the fluid blocker engages the lower distal nozzle housing;
FIG. 23 depicts a perspective view of an eighth exemplary alternative nozzle and a ninth exemplary fluid blocker, where the fluid blocker is disposed between a proximal nozzle housing and the upper and lower distal nozzle housings of the nozzle;
FIG. 23A depicts an enlarged cross-sectional view of a portion of the nozzle and the fluid blocker of FIG. 23, taken along line 23A-23A of FIG. 23;
FIG. 24A depicts a schematic cross-sectional view of a fifth exemplary alternative shaft assembly that includes a ninth exemplary alternative nozzle and a tenth exemplary fluid blocker, where a closure tube of the shaft assembly is in a proximal position causing an end effector to be in an open configuration;
FIG. 24B depicts a schematic cross-sectional view of the shaft assembly of FIG. 24A, where the closure tube of the shaft assembly is in a distal position causing the end effector to be in a closed configuration;
FIG. 25 depicts a schematic cross-sectional view of the shaft assembly of FIG. 24A taken along line 25-25 of FIG. 24A;
FIG. 26A depicts a schematic cross-sectional view of a sixth exemplary alternative shaft assembly that includes a tenth exemplary alternative nozzle and an eleventh exemplary fluid blocker, where a closure tube of the shaft assembly is in a proximal position causing an end effector to be in an open configuration;
FIG. 26B depicts a schematic cross-sectional view of the shaft assembly of FIG. 26A, where the closure tube of the shaft assembly is in a distal position causing the end effector to be in a closed configuration;
FIG. 27 depicts a bottom view of an eleventh exemplary alternative nozzle and a twelfth exemplary fluid blocker that rotatably engages with a distal end portion of the nozzle;
FIG. 28 depicts a front view of the fluid blocker of FIG. 27;
FIG. 29 depicts a perspective view of the fluid blocker of FIG. 27; and
FIG. 30 depicts another perspective view of the fluid blocker of FIG. 27.

The drawings are not intended to be limiting in any way, and it is contemplated that various embodiments of the invention may be carried out in a variety of other ways, including those not necessarily depicted in the drawings. The accompanying drawings incorporated in and forming a part of the specification illustrate several aspects of the present invention, and together with the description serve to explain the principles of the invention; it being understood, however, that this invention is not limited to the precise arrangements shown. The invention is defined by independent claim 1.

### DETAILED DESCRIPTION

The following description of certain examples of the invention should not be used to limit the scope of the present invention. Other examples, features, aspects, embodiments, and advantages of the invention will become apparent to those skilled in the art from the following description, which is by way of illustration, one of the best modes contemplated for carrying out the invention. As will be realized, the invention is capable of other different and obvious aspects, all without departing from the invention. Accordingly, the drawings and descriptions should be regarded as illustrative in nature and not restrictive.

For clarity of disclosure, the terms "proximal" and "distal" are defined herein relative to a surgeon, clinician, or other operator, grasping a surgical instrument having a distal surgical end effector. The term "proximal" refers to the position of an element arranged closer to the surgeon, and the term "distal" refers to the position of an element arranged closer to the surgical end effector of the surgical instrument and further away from the surgeon. Moreover, to the extent that spatial terms such as "upper," "lower," "vertical," "horizontal," or the like are used herein with reference to the drawings, it will be appreciated that such terms are used for exemplary description purposes only and are not intended to be limiting or absolute. In that regard, it will be understood that surgical instruments such as those disclosed herein may be used in a variety of orientations and positions not limited to those shown and described herein.

As used herein, the terms "about" and "approximately" for any numerical values or ranges indicate a suitable dimensional tolerance that allows the part or collection of components to function for its intended purpose as described herein.

### I. Exemplary Surgical Stapling Instrument

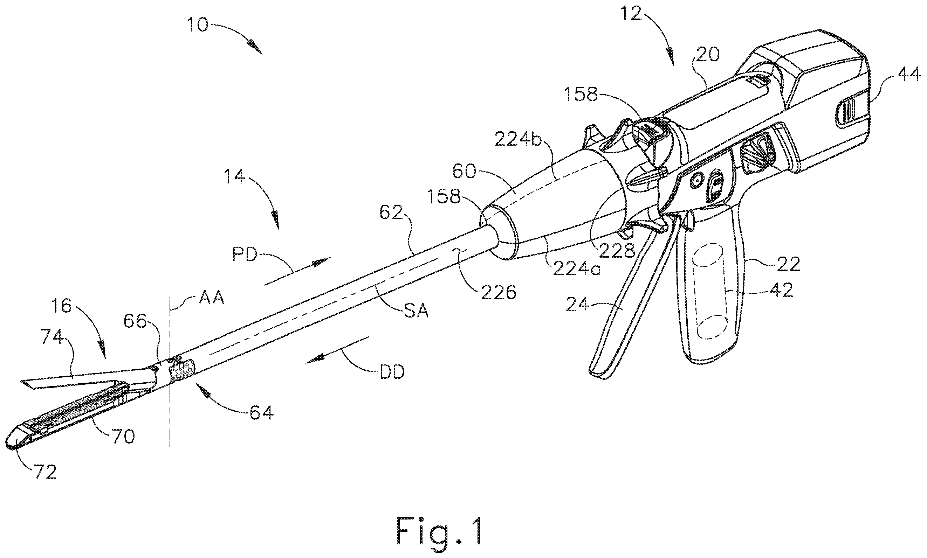

FIGS. 1-2 show a motor-driven surgical instrument (10) suitable for use in a variety of surgical procedures. In the illustrated example, instrument (10) includes a handle assembly (12) and an interchangeable shaft assembly (14) releasably coupled to and extending distally from handle assembly (12). Interchangeable shaft assembly (14) includes a surgical end effector (16) arranged at a distal end thereof, and which is configured to perform one or more surgical tasks or procedures. In some applications, interchangeable shaft assembly (14) may be effectively employed with a tool drive assembly of a robotically controlled or automated surgical system. For example, interchangeable shaft assembly (14) may be employed with various robotic systems, instruments, components, and methods such as those disclosed in U.S. Pat. No. 9,072,535, entitled "Surgical Stapling Instruments With Rotatable Staple Deployment Arrangements," issued July 7, 2015, the disclosure of which is referred to herein.

### A. Handle Assembly of Surgical Stapling Instrument

Handle assembly (12) comprises a body (20) that includes a pistol grip (22) configured to be grasped by a clinician, and a closure trigger (24) configured to pivot toward and away from pistol grip (22) to selectively close and open end effector (16), as described in greater detail below. In the present example, end effector (16) is configured to cut and staple tissue captured by end effector (16). In other examples, end effector (16) may be configured to treat tissue via application of various other types of movements and energies, such as radio frequency (RF) energy and/or ultrasonic energy, for example.

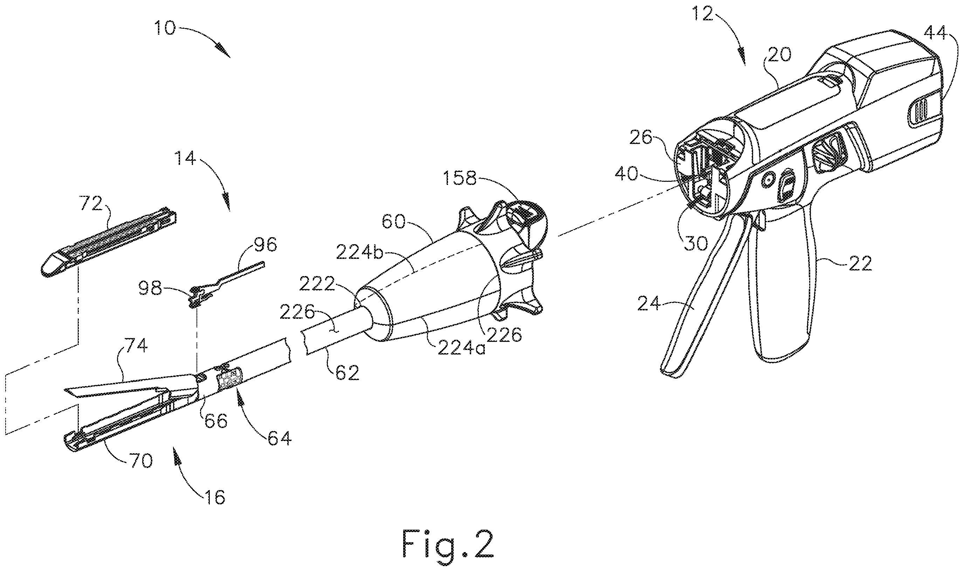

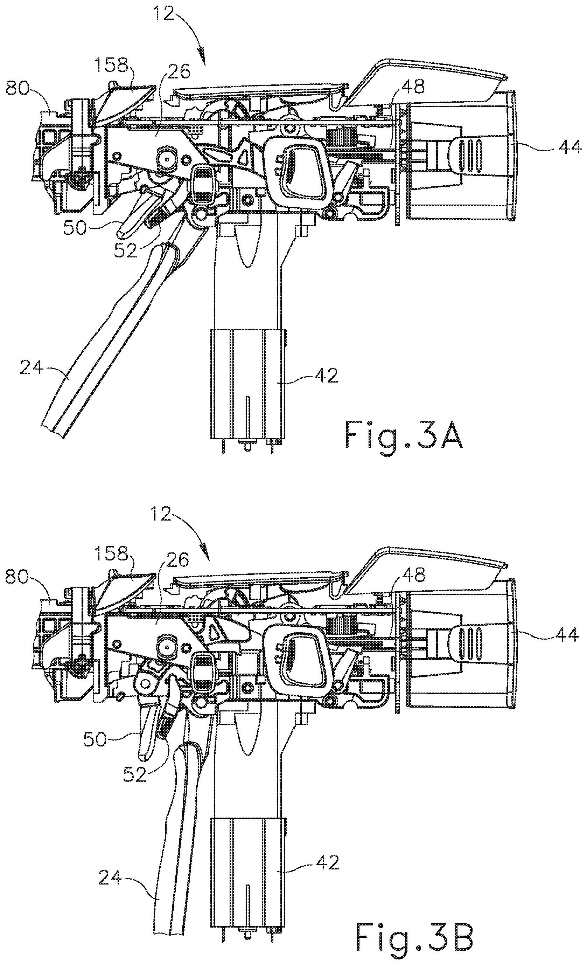

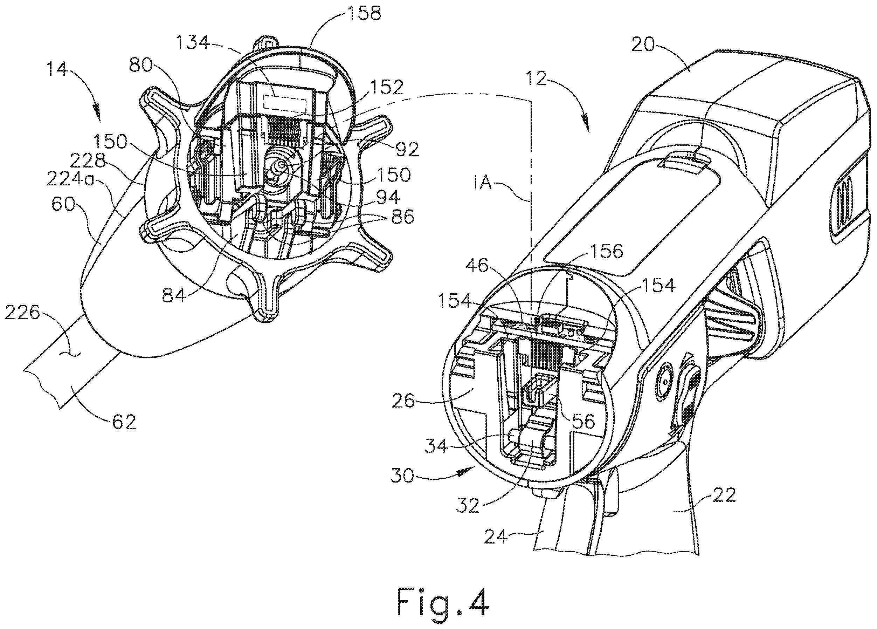

As seen in FIGS. 2-4, body (20) houses a support structure in the form of a handle frame (26) that supports a plurality of drive systems configured to generate and apply various control motions to corresponding portions of interchangeable shaft assembly (14). In particular, handle frame (26) supports a first drive system in the form of a closure drive system (30) that is operable to selectively close and open end effector (16) to thereby capture and release tissue. Closure drive system (30) includes an actuator in the form of closure trigger (24), which is pivotally supported by handle frame (26) and is operatively coupled with end effector (16) via components of shaft assembly (14) described below. Closure trigger (24) is configured to be squeezed by a clinician toward pistol grip (22) from an unactuated position (FIG. 3A) that provides end effector (16) in an open state for releasing tissue, to an actuated position (FIG. 3B) that provides end effector (16) in a closed state for clamping tissue. Closure trigger (24) may be biased toward the unactuated position by a resilient member (not shown). As seen best in FIG. 4, closure drive system (30) further comprises a linkage assembly that couples closure trigger (24) with end effector (16). The linkage assembly includes a closure link (32) and a transversely extending attachment pin (34) coupled to a distal end of closure link (32). Attachment pin (34) and the distal end of closure link (32) are accessible through a distal opening in handle assembly (12).

Handle assembly body (20) further supports a second drive system in the form of a firing drive system (40) (shown in FIG. 2) configured to apply firing motions to corresponding portions of interchangeable shaft assembly (14) and its end effector (16). In the present example, firing drive system (40) employs an electric motor (42) that is housed within pistol grip (22) of handle assembly (12) and is operatively coupled with end effector (16), as described below. Electric motor (42) may be of any suitable type, such as a DC brushed motor, a brushless motor, a cordless motor, a synchronous motor, a stepper motor, or any other suitable type of electric motor. Electric motor (42) is powered by a power source shown in the form of a power pack (44) removably coupled to a proximal portion of handle assembly body (20). Power pack (44) includes one or more batteries (not shown) of any suitable type, and batteries may be rechargeable or replaceable.

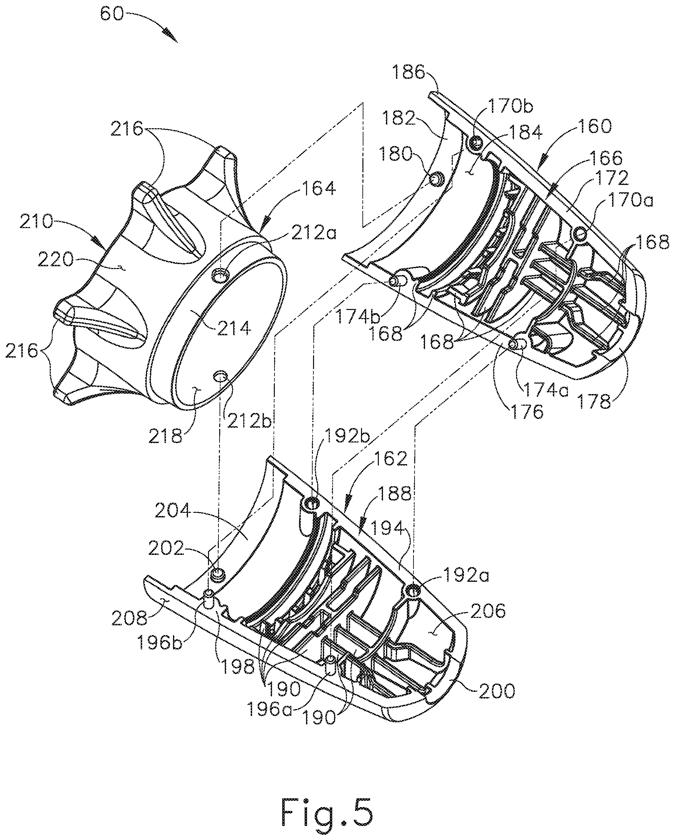

As seen in FIG. 4, electric motor (42) is electrically coupled to and controlled by a circuit board (46) supported by handle frame (26) within handle assembly body (20). Circuit board (46) may include a microcontroller and is configured to direct power from power pack (44) to electric motor (42) and thereby energize motor (42) to fire end effector (16). Electric motor (42) is configured to interface with a drive gear arrangement (not shown) that is operable to actuate an elongate drive member (48) axially relative to handle frame (26) in response to activation of motor (42). As seen best in FIG. 5, a distal end of drive member (48) is exposed through a distal opening of handle assembly (12) and is configured to couple to a translating member of shaft assembly (14) to thereby operatively couple motor (42) with end effector (16), as described below.

Electric motor (42) is energized by battery pack (44) in response to actuation of a firing trigger (50), which is pivotally supported by handle assembly (12) as best seen in FIGS. 3A and 3B. In the present example, firing trigger (50) is positioned "outboard" of closure trigger (24). Similar to closure trigger (24), firing trigger (50) is configured to be squeezed by the clinician toward pistol grip (22) from an unactuated position (FIG. 3B) to an actuated position (not shown). Firing trigger (50) may be biased toward the unactuated position by a resilient member (not shown). When firing trigger (50) is depressed from the unactuated position to the actuated position, firing trigger (50) causes battery pack (44) to energize motor (42) to actuate drive member (48) longitudinally and thereby fire end effector (16). As shown in FIGS. 3A and 3B, handle assembly (12) further includes a firing trigger safety button (52) that is selectively pivotable between a safety position and a firing position to prevent inadvertent actuation of firing trigger (50).

### B. Interchangeable Shaft Assembly of Surgical Stapling Instrument

As shown in FIGS. 1-2, interchangeable shaft assembly (14) of the present example includes a nozzle (60), a closure tube (62) extending distally from nozzle (60), an articulation joint (64) disposed at a distal end of closure tube (62), a distal closure tube segment (66) coupled to a distal end of articulation joint (64), and end effector (16) extending distally therefrom.

End effector (16) includes a first jaw comprising an elongate channel (70) that receives a cartridge (72), and a second jaw comprising an anvil (74) configured to pivot relative to channel (70) between open and closed positions for clamping tissue between anvil (74) and cartridge (72). Cartridge (72) is shown in the form of a conventional staple cartridge having features described in greater detail below, and is configured to fire a plurality of staples into tissue clamped by end effector (16). In other examples, end effector (16) may be suitably configured to apply a variety of other types of motions and energies to tissue captured by end effector (16), such as radio frequency (RF) energy and/or ultrasonic energy, for example. For instance, cartridge (72) may be configured to apply RF to tissue as generally disclosed in U.S. Pub. No. 2019/0000478, entitled "Surgical System Couplable With Staple Cartridge And Radio Frequency Cartridge, And Method Of Using Same," published January 3, 2019, the disclosure of which is referred to herein.

Anvil (74) of end effector (16) is operatively coupled with closure drive system (30) of handle assembly (12), and is configured to pivot between open and closed positions, about a pivot axis that extends transversely to shaft axis (SA), in response to actuation of closure trigger (24). In particular, anvil (74) is configured to assume an open position when closure trigger (24) is in the unactuated position, and a closed position when closure trigger (24) is depressed to the actuated position. Anvil (74) is coupled with closure drive system (30) via closure tube (62) and distal closure tube segment (66), among other components described below. Closure tube (62) and distal closure tube segment (66) are configured to translate proximally and distally relative to nozzle (60) to thereby actuate anvil (74) about its pivot axis in response to actuation of closure trigger (24).

Articulation joint (64) is configured to provide articulation of end effector (16) relative to closure tube (62) and corresponding components of shaft assembly (14) about an articulation axis (AA) that extends transversely to shaft axis (SA). In some examples, end effector (16) may be articulated to a desired orientation by pushing end effector (16) against soft tissue and/or bone within the patient. In other examples, end effector (16) may be articulated by an articulation driver (not shown).

As best seen in FIG. 4, nozzle (60) of interchangeable shaft assembly (14) houses a support structure in the form of a tool chassis (80) that rotatably supports nozzle (60). Nozzle (60) and end effector (16) are configured to rotate relative to tool chassis (80) about shaft axis (SA), as indicated in FIG. 1. Tool chassis (80) further supports a closure shuttle (84) that is configured to translate proximally and distally relative to tool chassis (80). A distal end of closure shuttle (84) is coupled to and rotatably supports a proximal end of closure tube (62). A proximal end of closure shuttle (84) includes a pair of proximally extending hooks (86) configured to couple with closure drive system (30) of handle assembly (12). In particular, hooks (86) are configured to releasably capture attachment pin (34) of closure drive system (30) when interchangeable shaft assembly (14) is coupled with handle assembly (12). Accordingly, actuation of closure trigger (24) to the actuated position (see FIG. 3B) drives closure shuttle (84) distally, which in turn drives closure tube (62) and distal closure tube segment (66) distally, thereby actuating anvil (74) to a closed position for clamping tissue with end effector (16). Returning trigger to the unactuated position (see FIG. 3A) actuates these components proximally, thereby returning anvil (74) to an open position.

As seen best in FIG. 4, interchangeable shaft assembly (14) further includes an internal firing system configured to operatively couple with firing drive system (40) of handle assembly (12) when shaft assembly (14) is coupled to handle assembly (12). Firing system includes an intermediate firing shaft (92) and closure tube (62). Intermediate firing shaft (92) includes a proximal end having an attachment lug (94) configured to rotatably seat within attachment cradle (56) of drive member (48) of firing drive system (40), and a distal end configured to couple to an elongate knife bar (96). Knife bar (96) is connected at its distal end to a knife member (98), which includes a sharpened cutting edge (99) configured to sever tissue clamped by end effector (16) as knife member advances distally through staple cartridge (72). Accordingly, actuation of firing trigger (50) actuates drive member (48) distally, which in turn drives intermediate firing shaft (92), knife bar (96), and knife member (98) distally to thereby cut tissue and simultaneously fire staple cartridge (72), as described below. Knife member (98) may include one or more anvil engagement features configured to engage and maintain anvil (74) in a closed state throughout cutting and stapling of tissue.

### C. Electrical Connections Within Surgical Instrument

Interchangeable shaft assembly (14) and variations thereof that are suitable for use with handle assembly (12) may employ one or more sensors and/or various other electrical components that require electrical communication with handle circuit board (46) of handle assembly (12). For instance, a proximal portion of shaft assembly (14) and/or end effector (16) may include one or more sensors and/or one or more RF electrodes (not shown) configured to electrically couple with handle circuit board (46) to enable operation thereof. As described below, shaft assembly (14) is suitably configured to enable rotation of end effector (16), among other components of shaft assembly (14), relative to handle assembly (12) while maintaining electrical coupling between shaft assembly (14) and handle assembly (12).

Interchangeable shaft assembly (14) may include a slip ring assembly (not shown) housed within nozzle (60). Slip ring assembly is configured to electrically couple shaft assembly (14) with handle assembly (12) for communication of electrical power and/or sensor signals between end effector (16) and handle circuit board (46). Slip ring assembly is configured to provide such electrical communication while facilitating rotation of nozzle (60) and end effector (16), among other rotating components of shaft assembly (14), relative to tool chassis (80) and handle assembly (12) about shaft axis (SA). Shaft circuit board (134), shown schematically in FIG. 4, may be mounted to shaft chassis (80) and include a microcontroller.

### D. Attachment of Interchangeable Shaft Assembly to Handle Assembly

As described in greater detail below, interchangeable shaft assembly (14) is configured to be releasably coupled with handle assembly (12). It will be appreciated that various other types of interchangeable shaft assemblies having end effectors configured for various types of surgical procedures may be used in combination with handle assembly (12) described above.

As shown best in FIG. 4, a proximal end of tool chassis (80) of interchangeable shaft assembly (14) includes a pair of tapered attachment members (150) extending transversely to shaft axis (SA), and a shaft-side electrical connector (152) positioned therebetween. For example, shaft-side electrical connector (152) and other shaft circuitry may be constructed and operable in accordance with at least some of the teachings of U.S. Pub. No. 2014/0263541, "Articulatable Surgical Instrument Comprising An Articulation Lock," published September 18, 2014 (now abandoned) and/or U.S. Pat. No. 9,913,642, entitled "Surgical System Comprising A Sensor System," issued March 13, 2018, the disclosures of which are referred to herein.

Shaft electrical connector (152) is in electrical communication with shaft circuit board (134) of shaft assembly (14). A distal end of handle frame (26) of handle assembly (12) includes a pair of dovetail receiving slots (154) and a handle-side electrical connector (156) arranged therebetween. Handle-side electrical connector (156) is in electrical communication with handle circuit board (46) of handle assembly (12). During attachment of shaft assembly (14) to handle assembly (12), as described below, tapered attachment members (150) are received within dovetail receiving slots (154) along an installation axis (IA) that is transverse to shaft axis (SA). Additionally, shaft electrical connector (152) is electrically coupled with handle-side electrical connector (156). The proximal end of interchangeable shaft assembly (14) additionally includes a latch assembly (158) configured to releasably latch tool chassis (80) to handle frame (26) of handle assembly (12) when shaft assembly (14) is coupled with handle assembly (12).

As shown in FIG. 4, to attach interchangeable shaft assembly (14) to handle assembly (12), the clinician first aligns tapered attachment members (150) of tool chassis (80) with dovetail receiving slots (154) of handle frame (26). The clinician then moves shaft assembly (14) toward handle assembly (12) along installation axis (IA), thereby seating tapered attachment members (150) within dovetail receiving slots (154) and lockingly engaging latch assembly (158) with a distal portion of handle assembly (12). In doing so, intermediate firing shaft (92) is seated within cradle (56) of longitudinally movable drive member (48), thereby operatively coupling firing system (90) of shaft assembly (14) with firing drive system (40) of handle assembly (12). Additionally, proximal hooks (86) of closure shuttle (84) slide over and capture opposed lateral ends of attachment pin (34) extending from closure link (32), thereby operatively coupling the anvil closure components of shaft assembly (14) with closure drive system (30) of handle assembly (12). Additionally, during attachment of shaft assembly (14) with handle assembly (12), shaft electrical connector (152) on tool chassis (80) is electrically coupled with handle-side electrical connector (156) on handle frame (26), thereby placing shaft circuit board (134) of shaft assembly (14) in electrical communication with handle circuit board (46) of handle assembly (12).

In various examples, surgical instrument (10) may be further configured in accordance with one or more teachings of U.S. Pat. No. 9,345,481, entitled "Staple Cartridge Tissue Thickness Sensor System," issued May 24, 2016; U.S. Pat. No. 8,608,045, entitled "Powered Surgical Cutting and Stapling Apparatus With Manually Retractable Firing System," issued December 17, 2013; U.S. Pat. Pub. No. 2019/0000465, entitled "Method For Articulating A Surgical Instrument," published January 3, 2019; U.S. Pat. Pub. No. 2019/0000464, entitled "Surgical Instrument With Axially Moveable Closure Member," published January 3, 2019; U.S. Pat. Pub. No. 2019/0000472, entitled "Surgical Instrument Comprising An Articulation System Lockable To A Frame," published January 3, 2019; U.S. Pat. No. 10,13 5,242, entitled "Smart Cartridge Wake Up Operation And Data Retention," issued November 20, 2018; U.S. Pat. No. 9,913,642, entitled "Surgical Instrument Comprising A Sensor System," issued March 13, 2018; U.S. Pat. Pub. No. 2014/0263552, entitled "Staple Cartridge Tissue Thickness Sensor System," published September 18, 2014 (now abandoned); and/or U.S. Pat. Pub. No. 2014/0263541, entitled "Articulatable Surgical Instrument Comprising An Articulation Lock," published September 18, 2014 (now abandoned), the disclosures of which are referred to herein.

### E. Nozzle

FIG. 5 shows an exploded perspective view of nozzle (60) of FIG. 1, where nozzle (60) includes an upper distal nozzle housing (160), a lower distal nozzle housing (162), and a proximal nozzle housing (164). Upper distal nozzle housing (160) includes a body (166). As shown, body (166) includes a plurality of ribs (168) configured to strengthen body (166). Body (166) also includes first and second recesses (170a-b) disposed on a first side (172) of body (166) and first and second projections (174a-b) disposed on a second side (176) of body (166). Body (166) also includes a semicircular recess (178) configured to receive a portion of closure tube (62). Body (166) includes a pin (180) disposed within a flange (182) of body (166) configured to couple with proximal nozzle housing (164). Body (166) includes inner and outer surfaces (184, 186).

Lower distal nozzle housing (162) is shown as being identical to upper distal nozzle housing (160). As such, lower distal nozzle housing (162) includes a body (188), where body (188) includes a plurality of ribs (190) configured to strengthen body (188). Body (188) also includes first and second recesses (192a-b) disposed on a first side (194) of body (188) and first and second projections (196a-b) disposed on a second side (198) of body (188). Body (188) also includes a semicircular recess (200) configured to receive another portion of closure tube (62), and a pin (202) disposed within a flange (204) of body (188) configured to couple with proximal nozzle housing (164). Body (188) includes inner and outer surfaces (206, 208).

Proximal nozzle housing (164) includes a body (210) that includes first and second recesses (212a-b) disposed on a flange (214). Body (210) also includes a plurality of outwardly extending fins (216). Outwardly extending fins (216) are spaced in an annular array. Body (210) includes inner and outer surfaces (218, 220). As shown using dashed lines in FIG. 5, first and second recesses (170a-b) disposed on a first side (172) of upper distal nozzle housing (160) couple with first and second projections (196a-b) of lower distal nozzle housing (162). Similarly, first and second projections (174a-b) of upper distal nozzle housing (160) couple with first and second recesses (192a-b) of lower distal nozzle housing (162). Semicircular recesses (178, 200) collectively form a 360-degree aperture (222) (shown in FIGS. 1-2) to receive closure tube (62).

### II. Exemplary Shaft Assemblies, Exemplary Nozzles, and Exemplary Fluid Blockers

Some versions of nozzle (60) may permit entry of fluid through nozzle (60) in some instances. Three fluid ingress methods are described; however, more or fewer are envisioned depending on the particular shaft assembly considered. First, fluid may enter nozzle (60) by travelling proximally along an outer surface (226) (shown in FIGS. 1-2) of closure tube (62) and into nozzle (60) between aperture (222) of nozzle (60) and outer surface (226) of closure tube (62). Secondly, fluid may also enter nozzle (60) within first and second longitudinally extending seams (224a-b) (shown in FIGS. 1-2) between upper and lower distal nozzle housings (160, 162). Thirdly, fluid may enter nozzle (60) within a perpendicularly extending seam (228) (shown in FIGS. 1-2) between flanges (182, 204) of upper and lower distal nozzle housings (160, 162) and flange (214) of proximal nozzle housing (164). Fluid ingress is undesirable because the interior of body assembly (shown as handle assembly (12)) may include moisture-sensitive electrical connections (e.g. to electrically connect sensors and/or electrical components contained within handle assembly (12) and/or external to handle assembly (12)). Fluid may adversely affect these moisture-sensitive electrical connections.

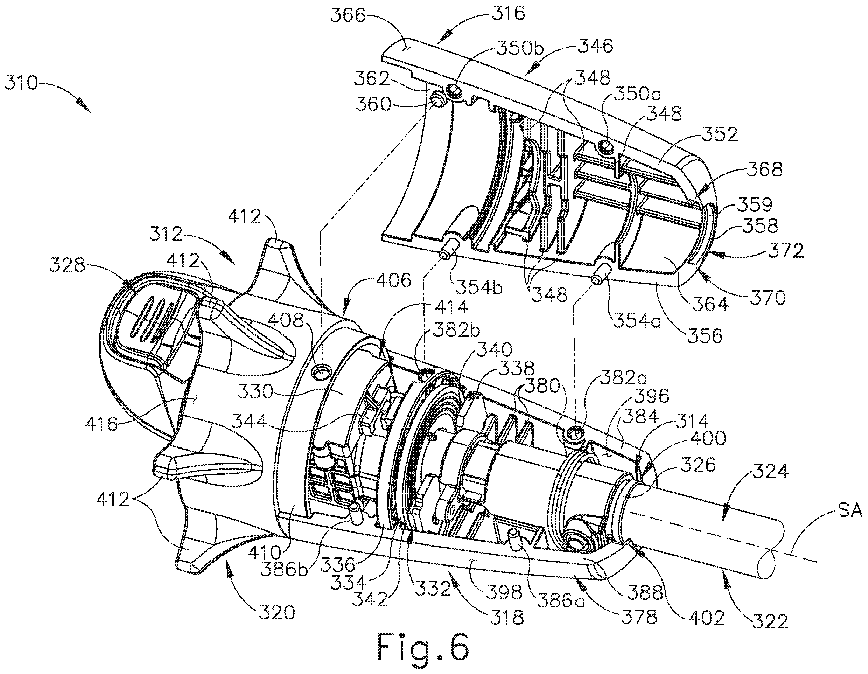

As a result, it is desirable to prevent, or at least minimize, fluid entering into nozzle (60) by incorporating one or more fluid blockers (314, 514, 614, 646, 714, 814, 914, 1014, 1114, 1214, 1314, 1414, 1514) into nozzles (312, 612, 712, 812, 912, 1012, 1112, 1212, 1312, 1412, 1512) as described below with reference to FIGS. 6-30. It is envisioned that multiple fluid blockers (314, 514, 614, 646, 714, 814, 914, 1014, 1114, 1214, 1314, 1414, 1514) may be used, and fluid blockers (314, 514, 614, 646, 714, 814, 914, 1014, 1114, 1214, 1314, 1414, 1514) may be used in combination with each other to further prevent or further minimize fluid from entering into nozzles (312, 612, 712, 812, 912, 1012, 1112, 1212, 1312, 1412, 1512).

As will be described in greater detail below with reference to FIGS. 6-30, surgical instrument (10) includes a handle assembly (12), a shaft assembly (310, 610, 710, 910, 1310, 1410), and an end effector (16). The handle assembly (e.g. handle assembly (12)) includes at least one electrical connection. End effector (16) is operable to treat tissue. Shaft assembly (310, 610, 710, 1310, 1410) extends between handle assembly (12) and end effector (16) along a shaft axis (SA). As previously described with reference to FIGS. 1-2, end effector (16) includes first and second opposing jaws. First jaw includes elongate channel (70) that is configured to receive staple cartridge (72). Second jaw includes anvil (74) configured to pivot relative to elongate channel (70) between open and closed positions for clamping tissue between anvil (74) and staple cartridge (72). As will be described in greater detail below, shaft assembly (310, 610, 710, 910, 1310, 1410) includes a nozzle (312, 612, 712, 812, 912, 1012, 1112, 1212, 1312, 1412, 1512) and one or more fluid blockers (314, 514, 614, 646, 714, 814, 914, 1014, 1114, 1214, 1314, 1414, 1514).

### A. First Exemplary Alternative Shaft Assembly with First Exemplary Alternative Nozzle and First Exemplary Fluid Blocker

FIGS. 6-9 show various views of a first exemplary alternative shaft assembly (310) that includes a first exemplary alternative nozzle (312) and a first exemplary fluid blocker (314), shown as an annular member. Shaft assembly (310) is similar to interchangeable shaft assembly (14) and nozzle (312) is similar to nozzle (60), except as where otherwise described. Nozzle (312) includes at least one housing. For example, nozzle (312) may include first and second distal housings (shown as upper and lower distal nozzle housings (316, 318)) and a proximal nozzle housing (320). Upper and lower distal nozzle housings (316, 318) are shown as being identical to one another; however, upper and lower distal nozzle housings (316, 318) may be different if desired. Upper and lower distal nozzle housings (316, 318) may be coupled together (e.g. snapped together) along with proximal nozzle housing (320) to form the housing of nozzle (312).

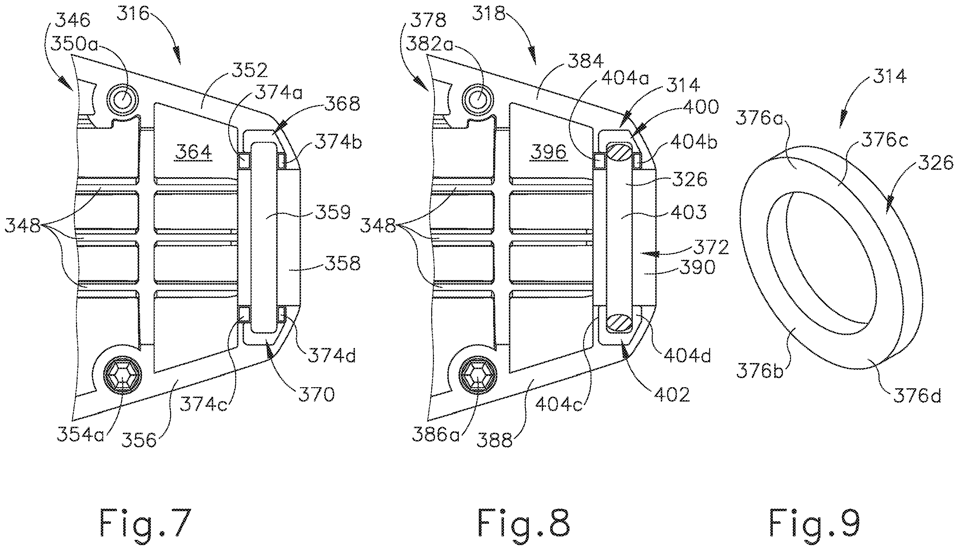

As shown in FIG. 6, fluid blocker (314) is disposed between upper and lower distal nozzle housings (316, 318) of nozzle (312). FIG. 7 shows a bottom enlarged view of the upper distal nozzle housing (316) of nozzle (312) of FIG. 6. Shaft assembly (310) includes a closure tube (322), similar to closure tube (62), which is configured to rotate about a shaft axis (SA) relative to a handle assembly (similar to handle assembly (12) or another suitable handle assembly). Closure tube (322) may be configured to translate relative to nozzle (312) along shaft axis (SA). As shown, fluid blocker (314) includes an annular body (326). As shown in FIG. 9, annular body (326) is integrally formed together as a unitary piece, and may be placed around an outer surface (324) of closure tube (322) prior to upper and lower distal nozzle housings (316, 318) being coupled together (e.g. at a nozzle press station). Annular body (326) of fluid blocker (314) may entirely surround closure tube (322). Annular body (326) of fluid blocker (314) is configured to wipe outer surface (324) of closure tube (322), as closure tube (322) moves longitudinally along shaft axis (SA). Similar to closure tube (62), closure tube (322) may move longitudinally proximally in a proximal direction (PD) (shown in FIG. 1) along shaft axis (SA), or distally in a distal direction (DD) (shown in FIG. 1) along shaft axis (SA).

FIG. 6 shows internal components of shaft assembly (310). As shown, shaft assembly (310) includes a latch assembly (328), similar to latch assembly (158), configured to releasably latch a tool chassis (330) (similar to tool chassis (80)) to handle frame (26) (shown in FIG. 2) of handle assembly (12) when shaft assembly (310) is coupled with handle assembly (12). Additionally, shaft assembly (310) includes a slip ring assembly (332), similar to slip ring assembly (120), which is housed within nozzle (312). Slip ring assembly (332) is configured to electrically couple shaft assembly (310) with handle assembly (12) for communication of electrical power and/or sensor signals between end effector (16) and handle circuit board (46). Slip ring assembly (332) is configured to provide such electrical communication while facilitating rotation of nozzle (312) and end effector (16), among other rotating components of shaft assembly (310), relative to tool chassis (330) and handle assembly (12) about shaft axis (SA). Slip ring assembly (332) comprises a proximal connector flange (334) mounted to a chassis flange (336) that extends distally from tool chassis (330) and a distal connector flange (338) secured to an interior of nozzle (312).

Distal connector flange (338) is configured to rotate with nozzle (312) relative to tool chassis (330) and chassis flange (336). Accordingly, the proximal face of distal connector flange (338) confronts and is configured to rotate relative to a distal face of proximal connector flange (334), about shaft axis (SA). The distal face of proximal connector flange (334) of slip ring assembly (332) includes a plurality of annular conductors (340) arranged substantially concentrically. The proximal face of distal connector flange (338) supports one or more electrical coupling members (342), each supporting a plurality of electrical contacts (not shown). Each electrical contact is positioned to contact a respective annular conductor (340) of proximal connector flange (334). Such an arrangement permits relative rotation between proximal connector flange (334) and distal connector flange (338) while maintaining electrical contact therebetween. Proximal connector flange (334) includes an electrical connector (344) extending proximally from a proximal face of proximal connector flange (334). Electrical connector (344) is configured to electrically couple annular conductors (340) with a shaft circuit board (not shown), but is similar to shaft circuit board (134), which may be mounted to tool chassis (80) and include a microcontroller.

As shown in FIGS. 6-7 and similar to upper distal nozzle housing (160), upper distal nozzle housing (316) includes a body (346). Body (346) includes a plurality of ribs (348) configured to strengthen body (346). Body (346) also includes first and second recesses (350a-b) disposed on a first side (352) of body (346) and first and second projections (354a-b) disposed on a second side (356) of body (346). Engagement features other than first and second recesses (350a-b) and first and second projections (354a-b) are also envisioned. More or fewer engagement features are also envisioned. As shown, body (346) also includes a semicircular recess (358) configured to receive a portion of closure tube (322). Body (346) also includes a pin (360) disposed within a flange (362) of body (346) configured to couple with proximal nozzle housing (320). Body (346) includes inner and outer surfaces (364, 366).

Unlike upper distal nozzle housing (160), upper distal nozzle housing (316) includes first and second retention features (368, 370) that are configured to retain annular body (326) within a recess (371) of a cavity (372) collectively formed by upper and lower distal nozzle housings (316, 318). As shown in FIG. 7, first retention feature (368) includes first and second opposing holders (374a-b) configured to receive a first portion (376a) of annular body (326) therebetween. Similarly, second retention feature (370) includes third and fourth opposing holders (374c-d) configured to receive a second portion (376b) of annular body (326) therebetween. First and second portions (376a-b) of annular body (326) are retained within recess (371) of cavity (372) by first, second, third, and fourth holders (374a-d).

As shown in FIGS. 6 and 8 and similar to lower distal nozzle housing (162), lower distal nozzle housing (318) includes a body (378). FIG. 8 shows an enlarged top view of fluid blocker (314) and lower distal nozzle housing (318) of FIG. 6, where a portion of fluid blocker (314) is disposed within lower distal nozzle housing (318). As shown, body (378) includes a plurality of ribs (380) configured to strengthen body (378). Body (378) includes first and second recesses (382a-b) disposed on a first side (384) of body (378) and first and second projections (386a-b) disposed on a second side (388) of body (378). Engagement features other than first and second recesses (382a-b) and first and second projections (386a-b) are also envisioned. More or fewer engagement features are also envisioned. As shown in FIG. 8, body (378) also includes a semicircular recess (390) configured to receive a portion of closure tube (322). Body (378) also includes a pin (not shown but similar to pin (360)) disposed within a flange (not shown but similar to flange (362)) of body (378) configured to couple with proximal nozzle housing (320). Body (378) includes inner and outer surfaces (396, 398).

Unlike lower distal nozzle housing (162), lower distal nozzle housing (318) includes first and second retention features (400, 402) that are configured to retain annular body (326) within a recess (403) of cavity (372). Cavity (372) is collectively formed by semicircular recesses (358, 390) and recesses (371, 403) of upper and lower distal nozzle housings (316, 318). Semicircular recesses (358, 390) and recesses (371, 403) may have the same or different shape and profile. As shown, first retention feature (400) includes first and second opposing holders (404a-b) configured to receive a third portion (376c) of annular body (326) therebetween. Similarly, second retention feature (370) includes third and fourth opposing holders (404c-d) configured to receive a fourth portion (376d) of annular body (326) therebetween. Third and fourth portions (376c-d) of annular body (326) are retained within recess (403) of cavity (372) by first, second, third, and fourth holders (376a-d). In other words, upper distal nozzle housing (316) of nozzle (312) captures upper portion (e.g. first and second portions (376a-b)) of annular body (326) within recess (371) of cavity (372), and lower distal nozzle housing (318) of nozzle (312) captures lower portion (e.g. third and fourth portions (376c-d)) of annular body (326) within recess (403) of cavity (372).

As shown in FIG. 6, proximal nozzle housing (320) is similar to proximal nozzle housing (164). Proximal nozzle housing (320) includes a body (406) that includes at least one recess (408) disposed on a flange (410). Body (406) also includes a plurality of outwardly extending fins (412). Outwardly extending fins (412) are spaced in an annular array. Body (406) includes inner and outer surfaces (414, 416). As shown, first and second recesses (408a-b) disposed on first side (352) of upper distal nozzle housing (316) couple with first and second projections (386a-b) of lower distal nozzle housing (318). Similarly, first and second projections (354a-b) of upper distal nozzle housing (316) couple with first and second recesses (382a-b) of lower distal nozzle housing (318).

FIG. 9 shows a perspective view of fluid blocker (314) of FIG. 6. As shown in FIG. 9, annular body (326) is integrally formed together as a unitary piece. Annular body (326) of fluid blocker (314) is disposed within recesses (371, 403) of cavity (372) of nozzle (312), where annular body (326) is configured to contact closure tube (322) to prevent fluid from entering nozzle (312) and reaching at least one electrical connection disposed within handle assembly (12). Annular body (326) is configured to provide a seal between closure tube (322) and nozzle (312) to prevent fluid from reaching at least one electrical connection disposed in handle assembly (12). For example, annular body (326) may be formed of an elastomeric material. Instead of or in addition to providing a seal between closure tube (322) and nozzle (312), annular body (326) may wick away fluid to prevent fluid from reaching at least one electrical connection disposed in handle assembly (12). Instead of or in addition to providing a seal between outer tube and nozzle or wicking fluid from closure tube (322), annular body (326) may be formed from a biocompatible fluid absorbing material that is configured to absorb fluid to prevent fluid from reaching at least one electrical connection disposed in handle assembly (12).

Shaft assembly (310) may provide many benefits including protecting internal components within shaft assembly (310) from potential fluid entering nozzle (312) (e.g. between closure tube (322) and upper and lower distal nozzle housings (316, 318)). For example, fluid blocker (314) may prevent, or at least minimize, fluid ingress in the space between closure tube (322) and semicircular recesses (358, 390) of upper and lower distal nozzle housings (160, 162). Additionally, shaft assembly (310) may include the similar internal shaft components as shaft assembly (14) and utilize a similar assembly process as shaft assembly (14) with minimal added components (e.g. fluid blocker (314)). Additionally, since the internal geometry of shaft assembly (310) accommodates fluid blocker (314) within recesses (371, 403) of cavity (372), this geometry modification of upper and lower distal nozzle housings (316, 318) may have the added benefit of stabilizing and/or centering closure tube (322) within nozzle (312). This may result in more consistent shifting and/or reduced interference of the switch collar with nozzle (312) while closure tube (322) is under load. Also, the interface gap between closure tube (322) and upper and lower distal nozzle housings (316, 318) of nozzle (312) may be reduced relative to nozzle (60).

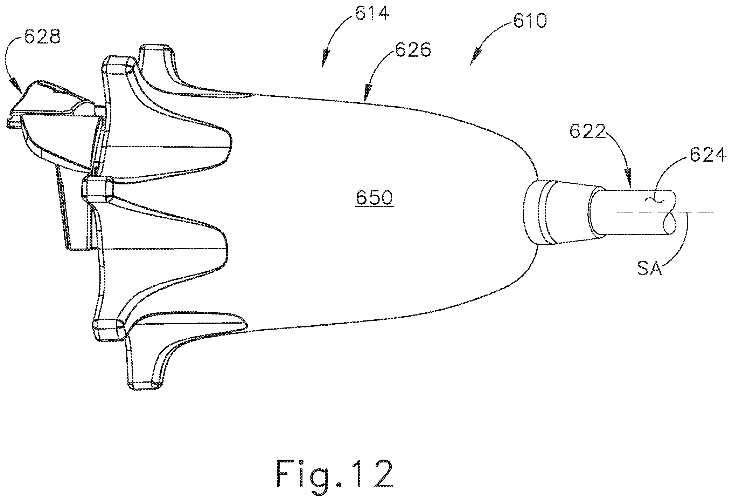

As will be described in greater detail below with reference to FIGS. 12-13A, nozzle (312) may optionally include a fluid blocker (614) coupled with outer surfaces (366, 398) of upper and lower distal nozzle housings (316, 318) to prevent fluid from reaching at least one electrical connection disposed within handle assembly (12). Fluid blocker (314) may be compressed between nozzle (312) and fluid blocker (614) described below. Inclusion of fluid blocker (614) is optional.

### B. Second Fluid Blocke According To The Claimed Invention

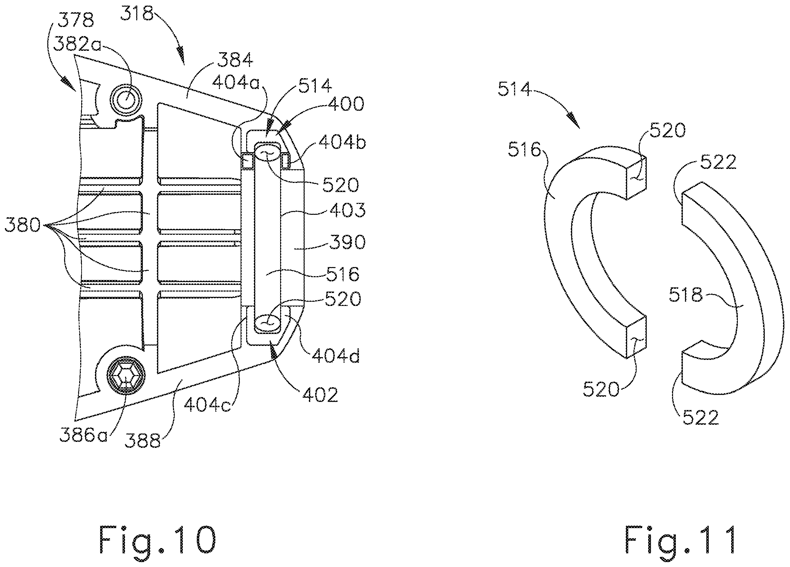

FIGS. 10-11 show a fluid blocker (514) according to the claimed invention, shown as a split annular member. FIG. 10 shows a top view of fluid blocker (514) disposed within recess (403) of cavity (372) of lower distal nozzle housing (318) of FIG. 6. FIG. 11 shows a perspective view of fluid blocker (514) of FIG. 10. As shown in FIGS. 10-11, fluid blocker (514) includes first and second portions (516, 518) that are completely separable from one another. Particularly, first and second portions (516, 518) each form about half of fluid blocker (514). However, it is envisioned that first and second portions (516, 518) may be different, with first or second portion (516, 518) comprising more than the other of first or second portion (516, 518). Splitting fluid blocker (514) into first and second portions (516, 518) may make assembly quicker and/or easier. First portion (516) is installed into recess (371) of upper distal nozzle housing (316), and second portion (518) is installed into recess (403) of lower distal nozzle housing (318) prior to upper and lower distal nozzle housings (316, 318) being coupled together as described above. End surfaces (520, 522) of first and second portions (516, 518) may include engagement features that couple together to prevent first portion (516) from moving relative to second portion (518) once installed.

### C. Second Exemplary Alternative Shaft Assembly with Second Exemplary Alternative Nozzle and Third Exemplary Fluid Blocker

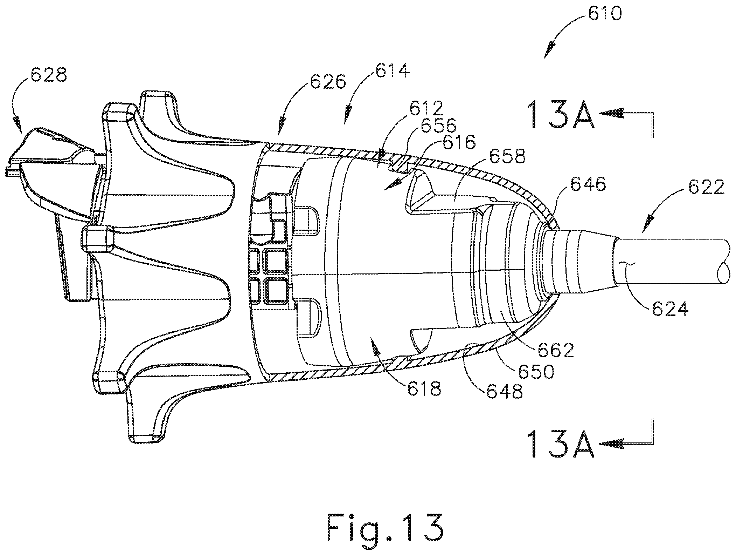

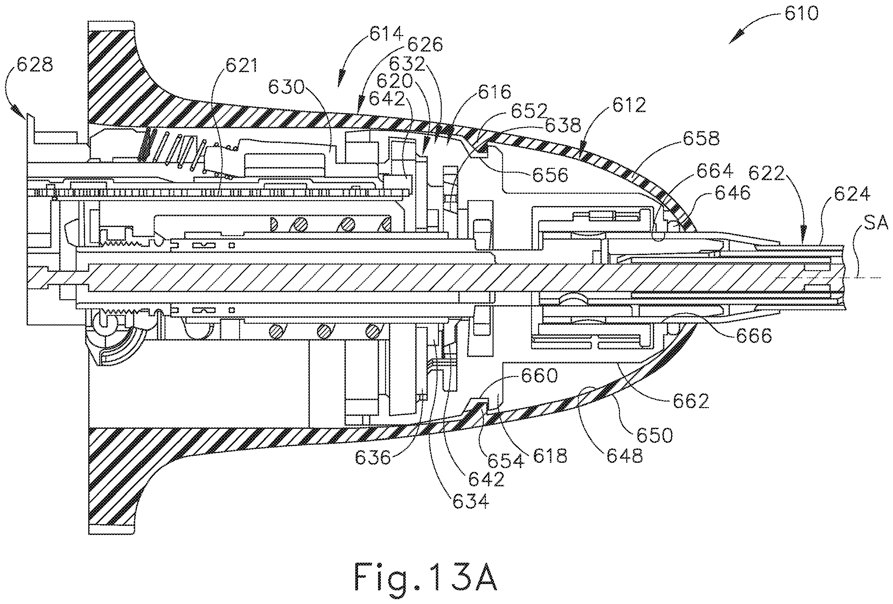

FIGS. 12-13 show a second exemplary alternative shaft assembly (610) that includes a second exemplary alternative nozzle (612) and a third exemplary fluid blocker (614). FIG. 13 shows a cross-sectional view of shaft assembly (610) of FIG. 12 taken along line 13-13 of FIG. 12. Shaft assembly (610) is similar to interchangeable shaft assembly (14), and nozzle (612) is similar to nozzle (60), except as where otherwise described below. As shown, fluid blocker (614) includes a monolithic outer body (626) that is coupled with upper and lower distal nozzle housings (616, 618).

Nozzle (612) includes at least one housing. For example, nozzle (612) may include first and second distal housings (shown as upper and lower distal nozzle housings (616, 618)). Shaft assembly (610) includes a closure tube (622), similar to closure tube (62), which is configured to rotate about a shaft axis (SA) relative to a handle assembly (e.g. handle assembly (12) or another suitable handle assembly). Closure tube (622) may be configured to translate relative to nozzle (612) along shaft axis (SA). Upper and lower distal nozzle housings (616, 618) may be pressed together using a variety of suitable methods. Upper and lower distal nozzle housings (616, 618) may contain and position internal components of shaft assembly (610). For example, these internal components of shaft assembly (610) may include a switch collar (620), a torsion spring, a sensor board (621), and a top cap. Similar to shaft assembly (14), shaft assembly (610) includes a latch assembly (628) (similar to latch assembly (158)), a tool chassis (630) (similar to tool chassis (80)), a slip ring assembly (632) (similar to slip ring assembly (120)), a proximal connector flange (634) (similar to proximal connector flange (122)), a chassis flange (636) (similar to chassis flange (126)), a distal connector flange (638) (similar to distal connector flange (124)), and one or more electrical coupling members (642) (similar to electrical coupling members (130)).

Monolithic outer body (626) of fluid blocker (614) includes inner and outer surfaces (648, 650). Monolithic outer body (626) may be over-molded onto upper and lower distal nozzle housings (616, 618). Alternatively, monolithic outer body (626) may be coupled with upper and lower distal nozzle housings (616, 618). Inner surface (648) of monolithic outer body (626) includes upper and lower coupling features (652, 654) that are configured to engage upper and lower distal nozzle housings (616, 618). As shown, an upper coupling feature (656) is disposed on an outer surface (658) of upper distal nozzle housing (616). Similarly, a lower coupling feature (660) is disposed on an outer surface (662) of lower distal nozzle housing (618). More or fewer coupling features are envisioned for fluid blocker (614) and upper and lower distal nozzle housings (616, 618). Upper and lower distal nozzle housings (616, 618) include semicircular recesses (664, 666).

Additionally, shaft assembly (610) may include a second fluid blocker (646). Second fluid blocker (646) may function similarly to fluid blocker (314) described above. Second fluid blocker (646) may be compressed between an outer nozzle (e.g. fluid blocker (614)) and an inner nozzle (e.g. upper and lower distal nozzle housings (616, 618)). For example, upper and lower coupling features (656, 660) of upper and lower distal nozzle housings (616, 618) securably engage upper and lower coupling features (652, 654) of monolithic outer body (626) and compress second fluid blocker (646) therebetween. Second fluid blocker (646) is shown as an annular member (e.g. an O-ring). Second fluid blocker (646) may be installed into monolithic outer body (626) against a distal inner face of monolithic outer body (626). Once second fluid blocker (646) is installed into monolithic outer body (626), this assembly may then be slid down closure tube (622) to securably engage upper and lower distal nozzle housings (616, 618). Fluid blocker (614) and second fluid blocker (646) prevent, or at least minimize, fluid from entering nozzle (612), without adding to the closure force to close first and second jaws together.

Shaft assembly (610) may provide many benefits. For example, by including fluid blocker (614) and/or second fluid blocker (646), internal components are protected by preventing, or at least reducing, fluid within shaft assembly (610) arising from ingress between closure tube (622) and upper and lower distal nozzle housings (616, 618) or between upper and lower distal nozzle housings (616, 618). As shown, monolithic outer body (626) of fluid blocker (614) has no seams that may allow for fluid ingress. Second fluid blocker (646) provides a seal around closure tube (622) that is compressed between fluid blocker (614) and upper and lower distal nozzle housings (616, 618). As a result, fluid blocker (614) and second fluid blocker (646) may reduce or altogether prevent fluid ingress in the space between closure tube (322) and semicircular recesses (358, 390) of upper and lower distal nozzle housings (616, 618). Additionally, various seal materials and geometries may be utilized as desired. Additionally, including fluid blocker (614) allows upper and lower distal nozzle housings (616, 618) to be designed with features and faces that allow easier manufacturing (e.g. pressing), since at least a portion of outer surfaces (658, 662) of upper and lower distal nozzle housings (616, 618) are not exposed, but instead, covered by outer surface (650) of monolithic outer body (626).

### D. Third Exemplary Alternative Shaft Assembly with Third Exemplary Alternative Nozzle and Fourth Exemplary Fluid Blocker

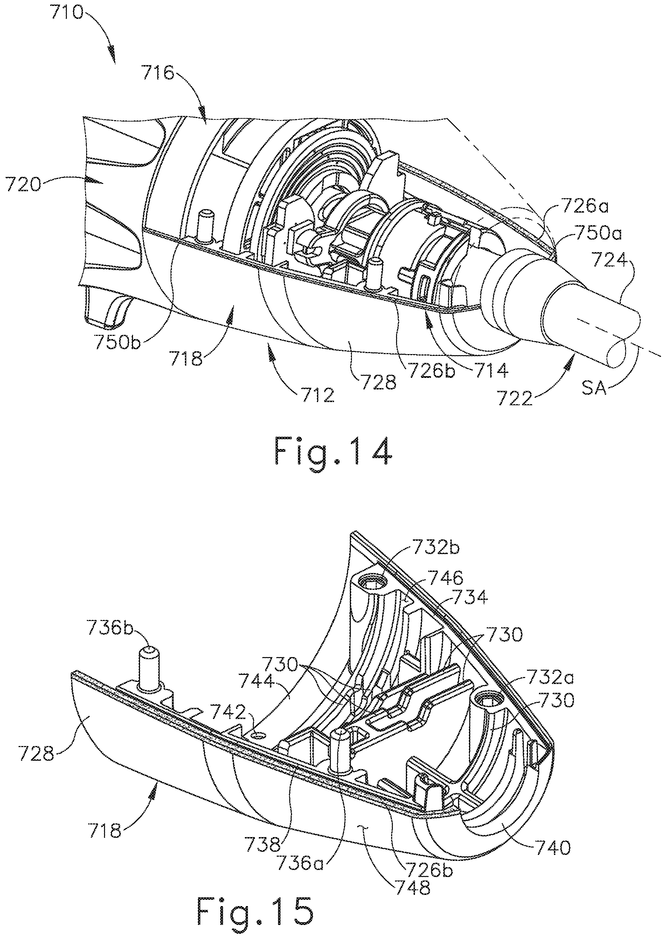

FIGS. 14-15 show perspective views of a third exemplary alternative shaft assembly (710) that includes a third exemplary alternative nozzle (712) and a fourth exemplary fluid blocker (714). Shaft assembly (710) is similar to interchangeable shaft assembly (14), and nozzle (712) is similar to nozzle (60), except as where otherwise described below. Nozzle (712) includes at least one housing. As shown, nozzle (712) includes an upper distal nozzle housing (716), a lower distal nozzle housing (718), and a proximal nozzle housing (720). Upper distal nozzle housing (716) may be similar to upper distal nozzle housing (160), lower distal nozzle housing (718) may be similar to lower distal nozzle housing (162), and proximal nozzle housing (720) may be similar to proximal nozzle housing (164).

As shown in FIG. 14, fluid blocker (714) is disposed between upper and lower distal nozzle housings (716, 718) of nozzle (712), with upper distal nozzle housing (716) being shown as transparent to reveal interior components. FIG. 15 shows a perspective view of lower distal nozzle housing (718) and fluid blocker (714) of FIG. 14. As shown, fluid blocker (714) includes first and second longitudinally extending seals (726a-b) that are coupled with nozzle (712). For example, first and second longitudinally extending seals (726a-b) may be over-molded onto nozzle (712), or first and second longitudinally extending seals (726a-b) may be formed from separate pieces that are subsequently coupled with (e.g. snapped onto) nozzle (712). As shown in FIGS. 14-15, first longitudinally extending seal (726a) is coupled with upper distal nozzle housing (716), and second longitudinally extending seal (726b) is coupled with lower distal nozzle housing (718). Shaft assembly (710) includes a closure tube (722), similar to closure tube (62), which is configured to rotate about a shaft axis (SA) relative to handle assembly (e.g. handle assembly (12) or another suitable handle assembly). Closure tube (722) may configured to translate relative to nozzle (712) along shaft axis (SA). Closure tube (722) includes an outer surface (724).

Lower distal nozzle housing (718) is described in greater detail with reference to FIG. 15. Lower distal nozzle housing (718) includes a body (728) (similar to body (188)). Body (728) includes a plurality of ribs (730) (similar to ribs (190)) that are configured to strengthen body (728). Body (728) also includes first and second recesses (732a-b) (similar to first and second recesses (192a-b)) disposed on a first side (734) of body (728). Body (728) includes first and second projections (736a-b) (similar to first and second projections (196a-b)) disposed on a second side (738) of body (728). Body (728) also includes a semicircular recess (740) (similar to semicircular recess (200)) configured to receive another portion of closure tube (722). Body (728) also includes a pin (742) disposed within a flange (744) of body (728) configured to couple with proximal nozzle housing (720). Body (728) includes inner and outer surfaces (746, 748). While lower distal nozzle housing (718) is shown as being identical to upper distal nozzle housing (716), upper and lower distal nozzle housings (716, 718) may vary. Upper and lower distal nozzle housings (716, 718) may be pressed together using a variety of suitable methods. Upper and lower distal nozzle housings (716, 718) may contain and position internal components of shaft assembly (710). Nozzle (712) includes first and second longitudinally extending seams (750a-b) disposed between upper and lower distal nozzle housings (716, 718).

Fluid blocker (714) prevents fluid from entering between upper and lower distal nozzle housings (716, 718) through first and second longitudinally extending seams (750a-b) defined by where the upper and lower distal nozzle housings (716, 718) meet. As shown, first and second longitudinally extending seals (726a-b) are formed from a compressible material that is compressed when upper and lower distal nozzle housings (716, 718) are pressed together. For example, the compressible material may be silicone or another suitable material. When upper and lower distal nozzle housings (716, 718) are pressed together (e.g. during assembly), the compressible material of first and second longitudinally extending seals (726a-b) are compressed to create a seal that prevents fluid from entering between first and second longitudinally extending seams (750a-b) of upper and lower distal nozzle housings (716, 718).

### E. Fourth Exemplary Alternative Nozzle and Fifth Exemplary Fluid Blocker

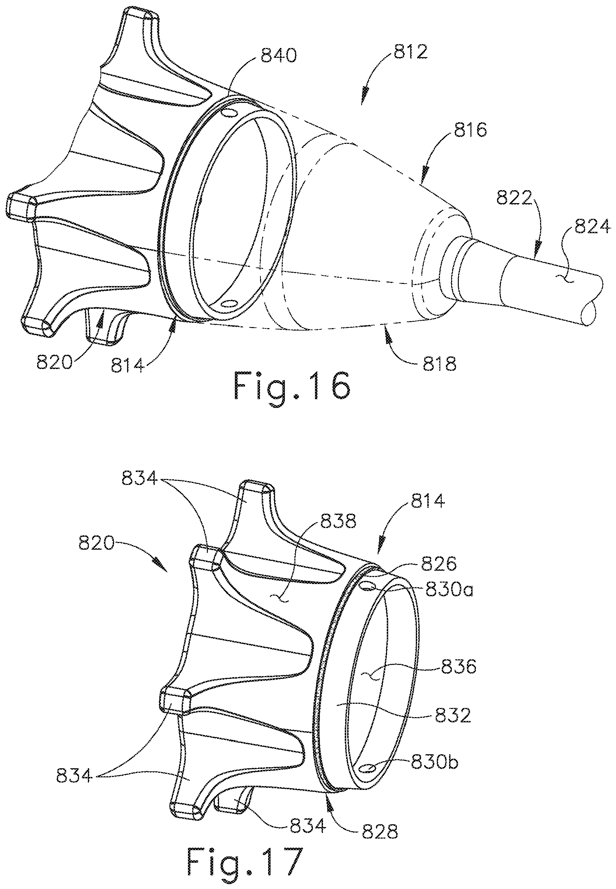

FIGS. 16-17 show perspective views of a fourth exemplary alternative nozzle (812) and a fifth exemplary fluid blocker (814). Nozzle (812) is similar to nozzle (60), except as where otherwise described below. Nozzle (812) includes at least one housing. As shown, nozzle (812) includes an upper distal nozzle housing (816), a lower distal nozzle housing (818), and a proximal nozzle housing (820). Upper distal nozzle housing (816) may be similar to upper distal nozzle housing (160), lower distal nozzle housing (818) may be similar to lower distal nozzle housing (162), and proximal nozzle housing (820) may be similar to proximal nozzle housing (164). A closure tube (822), similar to closure tube (62), is configured to rotate about shaft axis (SA) relative to a handle assembly (e.g. handle assembly (12) or another suitable handle assembly). Closure tube (822) may be configured to translate relative to nozzle (812) along shaft axis (SA). Closure tube (822) includes an outer surface (824).

FIG. 16 shows a perspective view where fluid blocker (814) is disposed between proximal nozzle housing (820) and upper and lower distal nozzle housings (816, 818). FIG. 17 shows a perspective view of fluid blocker (814) and proximal nozzle housing (820) of FIG. 16, where fluid blocker (814) is coupled with proximal nozzle housing (820). Proximal nozzle housing (820) includes a body (828) that includes first and second recesses (830a-b) disposed on a flange (832). Body (828) also includes a plurality of outwardly extending fins (834). Outwardly extending fins (834) are spaced in an annular array. Body (828) includes inner and outer surfaces (836, 838). As shown, fluid blocker (814) includes a perpendicularly extending seal (826) that is coupled with proximal nozzle housing (820). For example, perpendicularly extending seal (826) may be over-molded onto flange (832) of proximal nozzle housing (820). Alternatively, perpendicularly extending seal (826) may be formed from separate pieces that are subsequently coupled with (e.g. snapped onto) flange (832) of proximal nozzle housing (820). For example, perpendicularly extending seal (826) may include an O-ring that is assembled onto a groove (not shown) of flange (832). While not shown, it is also envisioned that perpendicularly extending seal (826) may be coupled with one or both of upper and lower distal nozzle housings (816, 818).

Fluid blocker (814) prevents fluid from entering through a perpendicularly extending seam (840) defined by where upper and lower distal nozzle housings (816, 818) meet proximal nozzle housing (820). As shown, perpendicularly extending seal (826) is formed from a compressible material that is compressed when upper and lower distal nozzle housings (816, 818) are pressed against proximal nozzle housing (820). For example, the compressible material may be a silicon or another suitable material. During assembly, when upper and lower distal nozzle housings (816, 818) are pressed together with proximal nozzle housing (820), the compressible material of perpendicularly extending seal (826) is compressed to form a seal that prevents fluid from entering through perpendicularly extending seam (840).

### F. Fourth Exemplary Alternative Shaft Assembly with Fifth Exemplary Alternative Nozzle and Sixth Exemplary Fluid Blocker

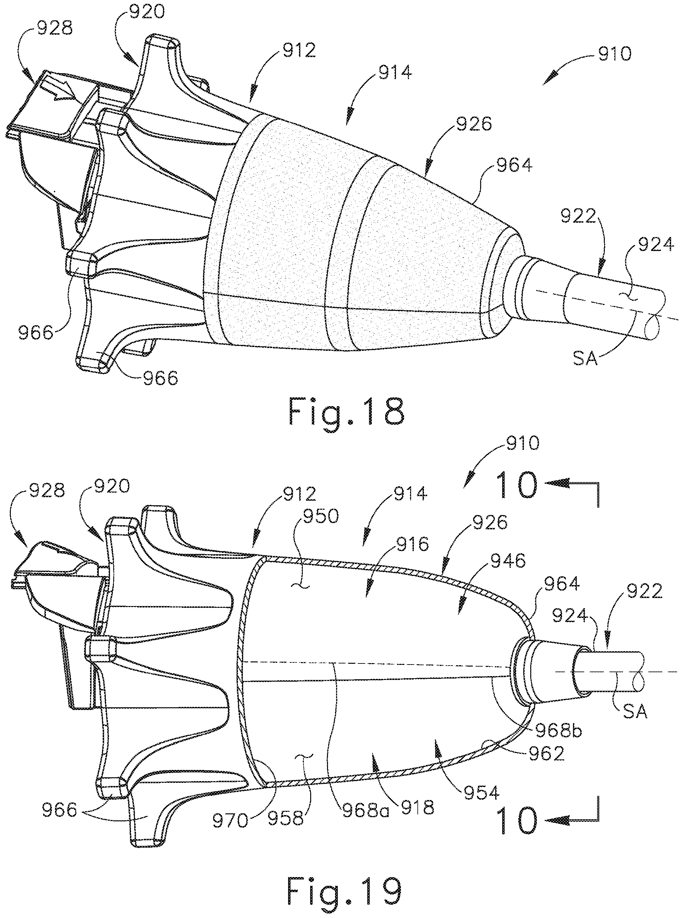

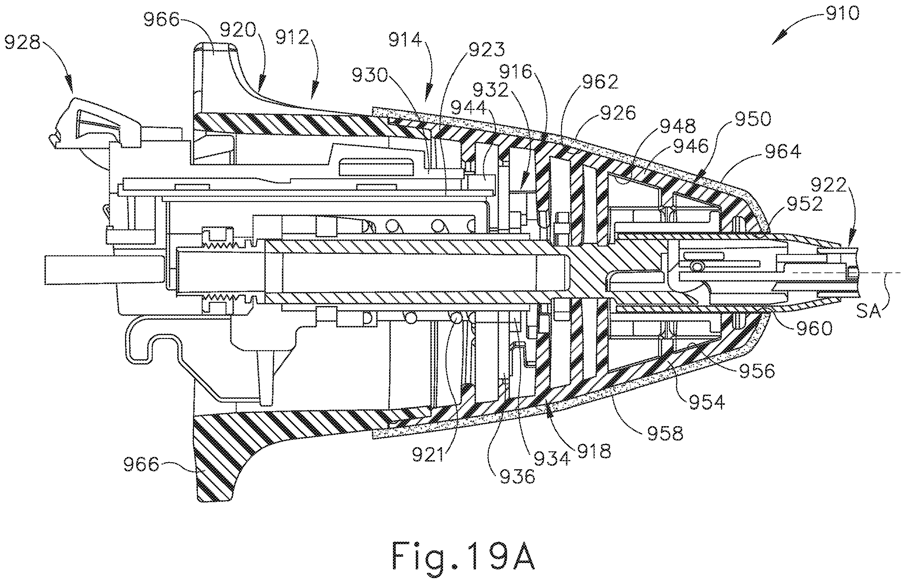

FIGS. 18-19A show various views of a fourth exemplary alternative shaft assembly (910) that includes a fifth exemplary alternative nozzle (912) and a sixth exemplary fluid blocker (914) covering at least a portion of nozzle (912). FIG. 19 shows a perspective view of shaft assembly (910) of FIG. 18, where fluid blocker (914) is shown as transparent. FIG. 19A shows a cross-sectional view of shaft assembly (910) of FIG. 19 taken along line 19A-19A of FIG. 19. Shaft assembly (910) is similar to interchangeable shaft assembly (14), and nozzle (912) is similar to nozzle (60), except as where otherwise described below. Nozzle (912) includes at least one housing. For example, nozzle (912) may include first and second distal housings (shown as upper and lower distal nozzle housings (916, 918)) and a proximal nozzle housing (920). As shown, fluid blocker (914) includes a monolithic outer body (926) that is coupled with nozzle (912). For example, monolithic outer body (926) may be over-molded onto, or otherwise surround, at least a portion of upper and lower distal nozzle housings (916, 918) and/or proximal nozzle housing (920).

Shaft assembly (910) includes a closure tube (922), similar to closure tube (62), which is configured to rotate about a shaft axis (SA) relative to a handle assembly (e.g. handle assembly (12) or another suitable handle assembly). Closure tube (922) may be configured to translate relative to nozzle (912) along shaft axis (SA). Upper and lower distal nozzle housings (916, 918) may be secured together using a variety of suitable methods (e.g. pressing). Upper and lower distal nozzle housings (916, 918) may contain and position internal components of shaft assembly (910). For example, these internal components of shaft assembly (910) may include a switch collar, a torsion spring (921), a sensor board (923), and a top cap. As shown in FIG. 19A and similar to shaft assembly (14), shaft assembly (910) includes a latch assembly (928) (similar to latch assembly (158)), a tool chassis (930) (similar to tool chassis (80)), a slip ring assembly (932) (similar to slip ring assembly (120)), a proximal connector flange (similar to proximal connector flange (122)), a chassis flange (936) (similar to chassis flange (126)), a distal connector flange (similar to distal connector flange (124)), one or more electrical coupling members (similar to electrical coupling members (130)), and an electrical connector (944) (similar to electrical connector (132)).

Upper nozzle housing (916) includes a body (946). Body (946) includes inner and outer surfaces (948, 950) and a semicircular recess (952). Similarly, lower nozzle housing (918) includes a body (954). Body (954) includes inner and outer surfaces (956, 958) and a semicircular recess (960). Monolithic outer body (926) of fluid blocker (914) includes inner and outer surfaces (962, 964). Monolithic outer body (926) of fluid blocker (914) prevents, or at least minimizes, fluid from entering nozzle (912) without adding to the closure force to close first and second jaws together. Monolithic outer body (926) may be assembled over outer surfaces (950, 958) of upper and lower distal nozzle housings (916, 918) and fins (966) of proximal nozzle housing (920) during manufacturing. Monolithic outer body (926) may eliminate a soft touch overmold on fins (966). Additionally, various seal materials and geometries may be utilized as desired.

Shaft assembly (910) may provide many benefits, such as protecting internal components by preventing, or at least reducing, fluid within shaft assembly (910). As shown, monolithic outer body (926) of fluid blocker (914) has no seams that may allow for fluid ingress. Monolithic outer body (926) seals the distal end of nozzle (912) between outer surface (924) of closure tube (922) and semicircular recesses (952, 960) of upper and lower distal nozzle housings (916, 918), first and second longitudinally extending seams (968a-b) between upper and lower distal nozzle housings (916, 918), and a perpendicularly extending seam (970) between upper and lower distal nozzle housings (916, 918) and proximal nozzle housing (920). Monolithic outer body (926) may block fluid from all entry points of nozzle (912). As a result, fluid blocker (914) may reduce or altogether prevent fluid ingress.

### G. Sixth Exemplary Alternative Nozzle and Seventh Exemplary Fluid Blocker

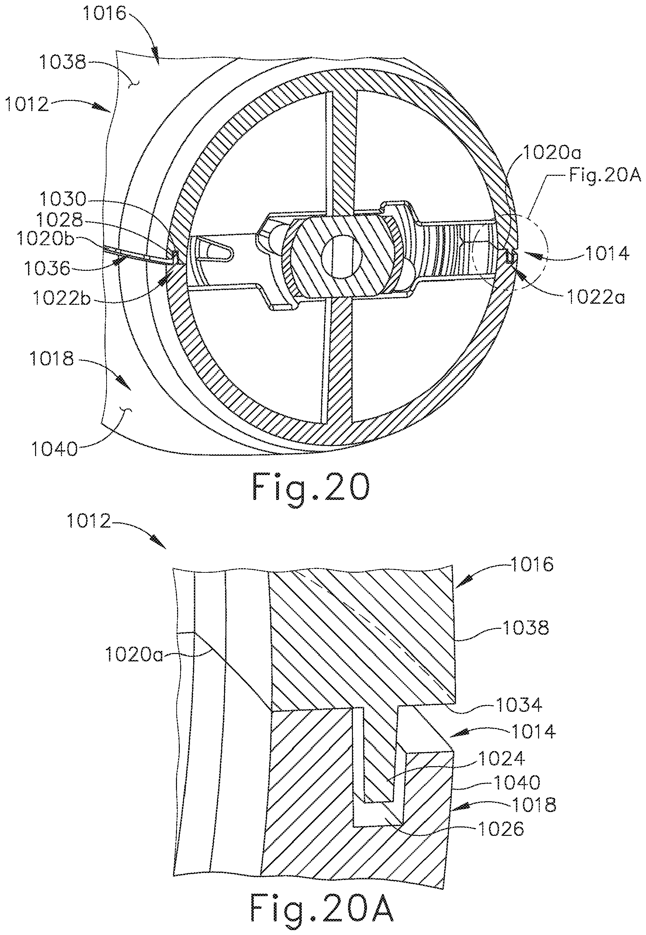

FIGS. 20-20A show perspective views of a sixth exemplary alternative nozzle (1012) and a seventh exemplary fluid blocker (1014). Nozzle (1012) includes at least one housing. For example, nozzle (1012) may include first and second distal housings (shown as upper and lower distal nozzle housings (1016, 1018)). As shown, upper and lower distal nozzle housings (1016, 1018) are identical, and may be coupled together (e.g. snapped together) to form nozzle (1012). FIG. 20A shows an enlarged portion of fluid blocker (1014) disposed between upper and lower distal nozzle housings (1016, 1018) of nozzle (1012) of FIG. 20. Fluid blocker (1014) uses interlocking features between upper and lower distal nozzle housings (1016, 1018) to create a tortuous path that prevents, or at least minimizes, fluid entering into the interior of nozzle (1012). For example, to seal first and second longitudinally extending seams (1020a-b), a tortuous fluid path may be created between first and second longitudinally extending seams (1020a-b).

As shown in FIG. 20, fluid blocker (1014) includes first and second tongue and groove assemblies (1022a-b). For first tongue and groove assembly (1022a), upper distal nozzle housing (1016) includes a projection (1024), i.e. a tongue, that tapers slightly inwardly. Lower distal nozzle housing (1018) includes a recess (1026), i.e. a groove, that is sized and configured to receive projection (1024). For second tongue and groove assembly (1022b), lower distal nozzle housing (1018) includes a projection (1028), i.e. a tongue, that tapers slightly inwardly. Upper distal nozzle housing (1016) includes a recess (1030), i.e. a groove, that is sized and configured to receive projection (1028). Since upper and lower distal nozzle housings (1016, 1018) alternate between projections (1024, 1028) and recesses (1026, 1030), upper and lower distal nozzle housings (1016, 1018) may be identical. Gaps (1034, 1036) may extend along outer surfaces (1038, 1040) of upper and lower distal nozzle housings (1016, 1018) for first and second tongue and groove assemblies (1022a-b). As shown, fluid blocker (1014) accounts for the geometries of upper and lower distal nozzle housings (1016, 1018) by alternating the side where first and second tongue and groove assemblies (1022a-b) are located, while still allowing for upper and lower distal nozzle housings (1016, 1018) to snap together.

Nozzle (1012) minimizes changes to nozzle architecture and assembly, with fluid blocker (1014) being added. Including first and second tongue and groove assemblies (1022a-b) of fluid blocker (1014) into first and second longitudinally extending seams (1020a-b) prevents, or at least minimizes, fluid from further migration into nozzle (1012). Fluid blocker (1014) may or may not hermetically seal nozzle (1012). It is desirable that fluid blocker (1014) cause enough of a blockage to prevent the vast majority of fluid from entering nozzle (1012). While not shown, a perpendicularly extending seam between upper and lower distal nozzle housings (1016, 1018) and proximal nozzle housing (not shown but similar to proximal nozzle housing (164)) may include a feature disposed around at least a portion of the circumference, or the entire circumference, mating upper and lower distal nozzle housings (1016, 1018) with the proximal nozzle housing.

### H. Seventh Exemplary Alternative Nozzle and Eighth Exemplary Fluid Blocker

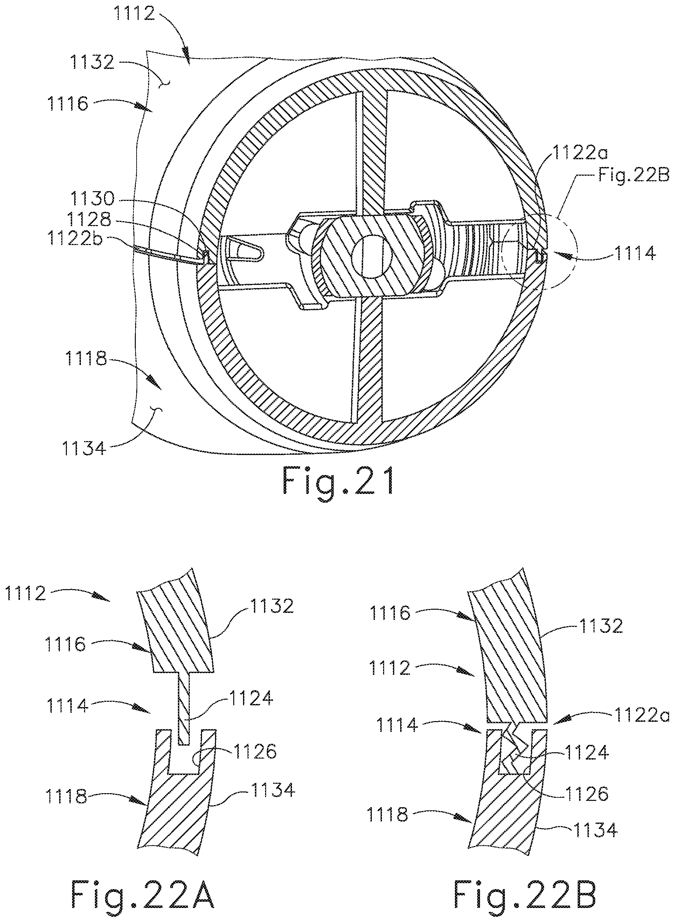

FIGS. 21-22B show various views of a seventh exemplary alternative nozzle (1112) and an eighth exemplary fluid blocker (1114), that unlike fluid blocker (1114) is at least partially deformable. Nozzle (1112) includes at least one housing. For example, nozzle (1112) may include first and second distal housings (shown as upper and lower distal nozzle housings (1116, 1118)). As shown, upper and lower distal nozzle housings (1116, 1118) are identical, and may be coupled together (e.g. snapped together) to form nozzle (1112). FIG. 21 shows fluid blocker (1114) disposed between upper and lower distal nozzle housings (1116, 1118) of nozzle (1112).

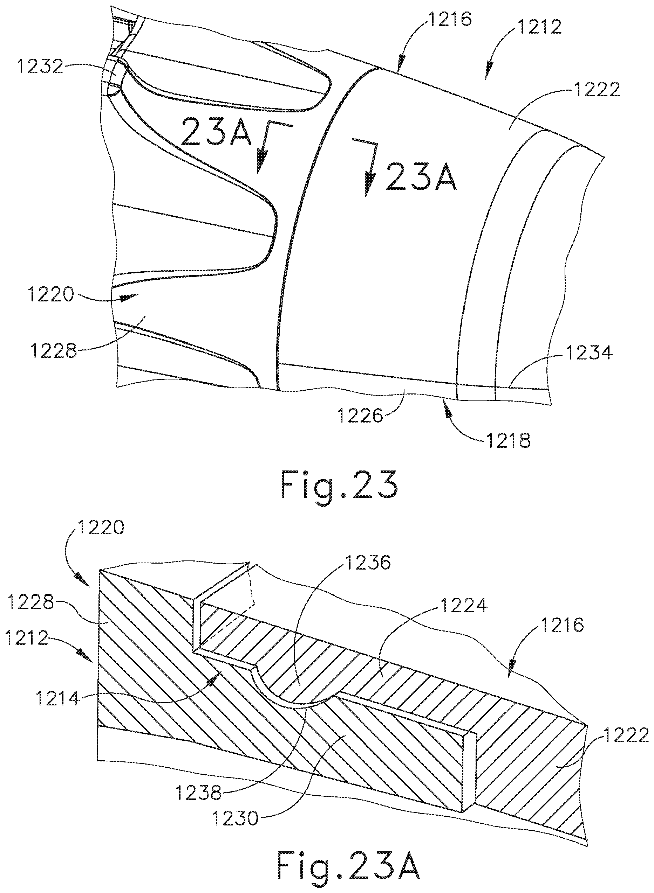

Fluid blocker (1114) uses interlocking deformable features between upper and lower distal nozzle housings (1116, 1118) to create a tortuous path that prevents, or at least minimizes, fluid entering into nozzle (1112). For example, to seal first and second longitudinally extending seams (1120a-b), a tortuous path may be created between first and second longitudinally extending seams (1120a-b). FIG. 22A shows an enlarged schematic sectional view of fluid blocker (1114) and upper and lower distal nozzle housings (1116, 1118) of FIG. 21, prior to fluid blocker (1114) engaging lower distal nozzle housing (1118). FIG. 22B shows enlarged portion of fluid blocker (1114) and upper and lower distal nozzle housings (1116, 1118) of FIG. 23A, but after a portion of fluid blocker (1114) engages lower distal nozzle housing (1118). As shown, fluid blocker (1114) includes first and second tongue and groove assemblies (1122a-b). For first tongue and groove assembly (1122a), upper distal nozzle housing (1116) includes a deformable projection (1124), i.e. a tongue, that tapers slightly inwardly. Lower distal nozzle housing (1118) includes a recess (1126), i.e. a groove, that is sized and configured to receive deformable projection (1124). As shown in FIG. 22B, deformable projection (1124) crushes during assembly, creating the tight seal. For second tongue and groove assembly (1122b) shown in FIG. 21, lower distal nozzle housing (1118) includes a deformable projection (1128), i.e. a tongue, that tapers slightly inwardly. Upper distal nozzle housing (1116) includes a recess (1130), i.e. a groove, that is sized and configured to receive deformable projection (1128). Deformable projections (1124, 1128) may have a variety of shapes and sizes.

Since upper and lower distal nozzle housings (1116, 1118) alternate between deformable projections (1124, 1128) and recesses (1126, 1130), upper and lower distal nozzle housings (1116, 1118) may be identical. For example, upper distal nozzle housing (1116) includes both deformable projection (1124) and recess (1126), and lower distal nozzle housing (1118) includes both deformable projection (1128) and recess (1130). As such, fluid blocker (1114) accounts for geometries of upper and lower distal nozzle housings (1116, 1118) by alternating the side where first and second tongue and groove assemblies (1122a-b) are located, while still allowing for upper and lower distal nozzle housings (1116, 1118) to snap together. Deformable projections (1124, 1128) deform when pressed together, minimizing potential issues around part tolerances while maintaining a sufficient seal. Part tolerances and geometry may be difficult to control. Fluid blocker (1114) allows for deformable projections (1124, 1128) to deform when pressed into recesses (1126, 1130) to create an extremely tight seal, while not requiring perfect part-to-part alignment during assembly. Upper and lower distal nozzle housings (1116, 1118) include outer surfaces (1038, 1040).

Nozzle (1112) minimizes changes to nozzle architecture and assembly, with few additional components being added (e.g. fluid blocker (1114)). Including first and second tongue and groove assemblies (1122a-b) of fluid blocker (1114) into first and second longitudinally extending seams (1120a-b) prevents, or at least minimizes, fluid from further migration into nozzle (1112). Fluid blocker (1114) may or may not hermetically seal nozzle (1112). It is desirable that fluid blocker (1114) cause enough of a blockage to prevent the vast majority of fluid from entering nozzle (1112). While not shown, a perpendicularly extending seam disposed between upper and lower distal nozzle housings (1116, 1118) and proximal nozzle housing may include a feature around at least a portion of the circumference, or the entire circumference, mating upper and lower distal nozzle housings (1116, 1118) with proximal nozzle housing.

### I. Eighth Exemplary Alternative Nozzle and Ninth Exemplary Fluid Blocker

FIG. 23-23A show perspective views of an eighth exemplary alternative nozzle (1212) and a ninth exemplary fluid blocker (1214). Nozzle (1212) is similar to nozzle (60), except as where otherwise described. Nozzle (1212) may include first and second distal housings (shown as upper and lower distal nozzle housings (1216, 1218)) and a proximal nozzle housing (1220). As shown, upper and lower distal nozzle housings (1216, 1218) are identical and may be coupled together (e.g. snapped together) to form nozzle (1212).

Upper distal nozzle housing (1216) includes a body (1222) (similar to body (166)). Body (1222) includes a flange (1224) similar to flange (182) shown in FIG. 5. Lower distal nozzle housing (1218) includes a body (1226), where body (1226) includes a flange (not shown) but similar to flange (204) shown in FIG. 5. Proximal nozzle housing (1220) includes a body (1228) that includes a flange (1230) (similar to flange (214)) and a plurality of outwardly extending fins (1232) (similar to fins (216)). Outwardly extending fins (1232) are spaced in an annular array. Similarly, perpendicularly extending seam (1234) extends between upper and lower distal nozzle housings (1216, 1218) and proximal nozzle housing (1220).

Fluid blocker (1214) is disposed between upper and lower distal nozzle housings (1216, 1218) and proximal nozzle housing (1220). FIG. 23A shows an enlarged portion of fluid blocker (1214) of FIG. 23. Fluid blocker (1214) may use interlocking features between upper and lower distal nozzle housings (1216, 1218) and proximal nozzle housing (1220) to create a tortuous path that prevents, or at least minimizes, fluid entering into nozzle (1212). For example, to seal perpendicularly extending seam (1234), a tortuous path may be created within perpendicularly extending seam (1234). Fluid blocker (1214) includes a projection (1236) and a recess (1238) that is sized and shaped to receive projection (1236). More specifically, flange (1224) of upper distal nozzle housing (1216) includes projection (1236) that is received by recess (1238) disposed in flange (1224) of proximal nozzle housing (1220). The interlocking features (e.g. projection (1236) and recess (1238)) of fluid blocker (1214) may be disposed around the entire circumference, or only along a portion of the circumference. For example, projection (1236) and recess (1238) may be annular to completely circumferentially seal nozzle (1212).

Nozzle (1212) minimizes changes to nozzle architecture and assembly procedures, with minimal components being added (e.g. fluid blocker (1214)). Including interlocking features (e.g. projection (1236) and recess (1238)) of fluid blocker (1214) into perpendicularly extending seam (1234) prevents, or at least minimizes, fluid from further migration into nozzle (1212). Fluid blocker (1214) may or may not hermetically seal nozzle (1212). It is desirable that fluid blocker (1214) cause enough of a blockage to prevent fluid from entering nozzle (1212). While not shown, fluid blocker (1214) may be a true ship lap between flange (1224) of upper distal nozzle housing (1216) and flange (1230) of proximal nozzle housing (1220). Similar to a true ship lap, the outer portion recess (1238) may be optional, and may allow for thicker sections while maintaining a sufficient seal.

### J. Fifth Exemplary Alternative Shaft Assembly with Ninth Exemplary Alternative Nozzle and Tenth Exemplary Fluid Blocker

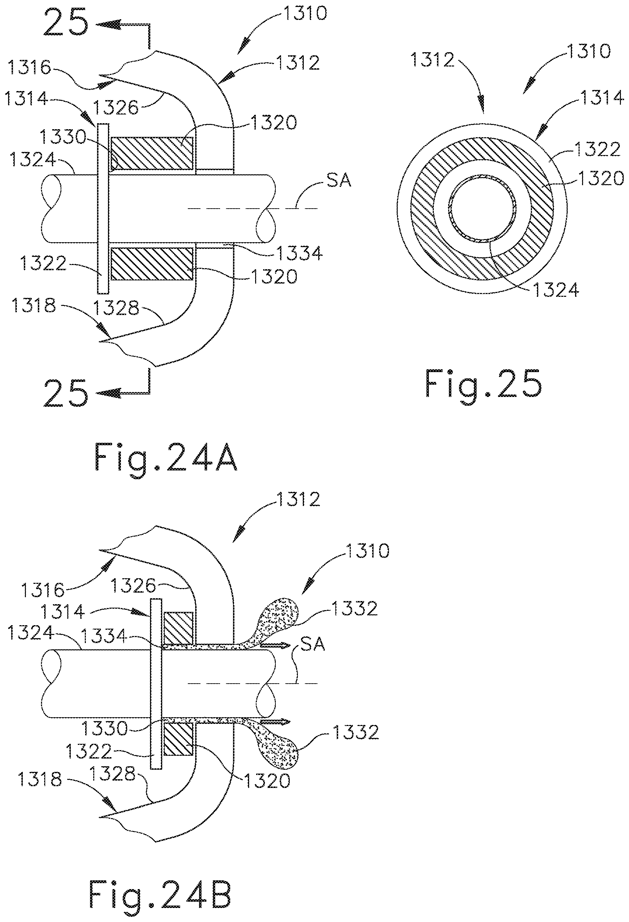

FIGS. 24A-25 show cross-sectional views of a fifth exemplary alternative shaft assembly (1310) that includes a ninth exemplary alternative nozzle (1312) and a tenth exemplary fluid blocker (1314). Nozzle (1312) may include at least one housing (shown as upper and lower distal nozzle housings (1316, 1318)). As shown, upper and lower distal nozzle housings (1316, 1318) are identical and may be coupled together (e.g. snapped together) to form nozzle (1312). Upper and lower distal nozzle housings (1316, 1318) may be different if desired. As shown, fluid blocker (1314) includes a fluid seal (1320) and a fluid shield (1322), where fluid shield (1322) is coupled with a closure tube (1324) (similar to closure tube (62)). Fluid seal (1320) and fluid shield (1322) may be annular.

FIG. 24A shows a schematic cross-sectional view of shaft assembly (1310), where closure tube (1324) of shaft assembly (1310) is in a proximal position causing an end effector (not shown, but similar to end effector (16)) to be in an open configuration. As shown in FIG. 24A, fluid seal (1320) is in a stretched or unstretched state and seals against inner surfaces (1326, 1328) of upper and lower distal nozzle housings (1316, 1318) and outwardly facing surface (1330) of fluid shield (1322). FIG. 24B shows a schematic sectional view of shaft assembly (1310) of FIG. 24A, where closure tube (1324) of shaft assembly (1310) is in a distal position causing the end effector (not shown, but similar to end effector (16)) to be in a closed configuration. As shown in FIG. 24B, fluid seal (1320) is in a compressed state that seals against inner surfaces (1326, 1328) of upper and lower distal nozzle housings (1316, 1318) and outwardly facing surface (1330) of fluid shield (1322). In the compressed state, fluid seal (1320) causes fluid (1332) to be pushed distally out of an annular cavity (1334). While FIGS. 24A-24B show closure tube (1324) as being a solid, monolithic piece of material, closure tube (1324) is intended to be hollow, as shown in FIG. 25.

FIG. 25 shows a schematic cross-sectional view of shaft assembly (1310) of FIG. 24A taken along line 25-25 of FIG. 24A. FIG. 25 shows closure tube (1324) as hollow and omits the components that are located within the hollow interior of closure tube (1324). These interior components of shaft assembly (1310) are also envisioned. Fluid seal (1320) may be a compressible cylindraceous seal (e.g. foam) that is interposed between fluid shield (1322) and upper and lower distal nozzle housings (1316, 1318) of nozzle (1312). Fluid seal (1320) may act as a spring to aid in opening the end effector (e.g. end effector (16)). Fluid shield (1322) may be a disc-shaped component that is fixedly secured to closure tube (1324), which translates longitudinally relative to upper and lower distal nozzle housings (1316, 1318) of nozzle (1312) to provide closure of anvil (not shown, but similar to anvil (74)). Fluid blocker (1314), shown as including fluid seal (1320) and fluid shield (1322), protect the interior of nozzle (1312) from fluid ingress without adding friction or drag to the opening or closing of the end effector.

### K. Sixth Exemplary Alternative Shaft Assembly with Tenth Exemplary Alternative Nozzle and Eleventh Exemplary Fluid Blocker

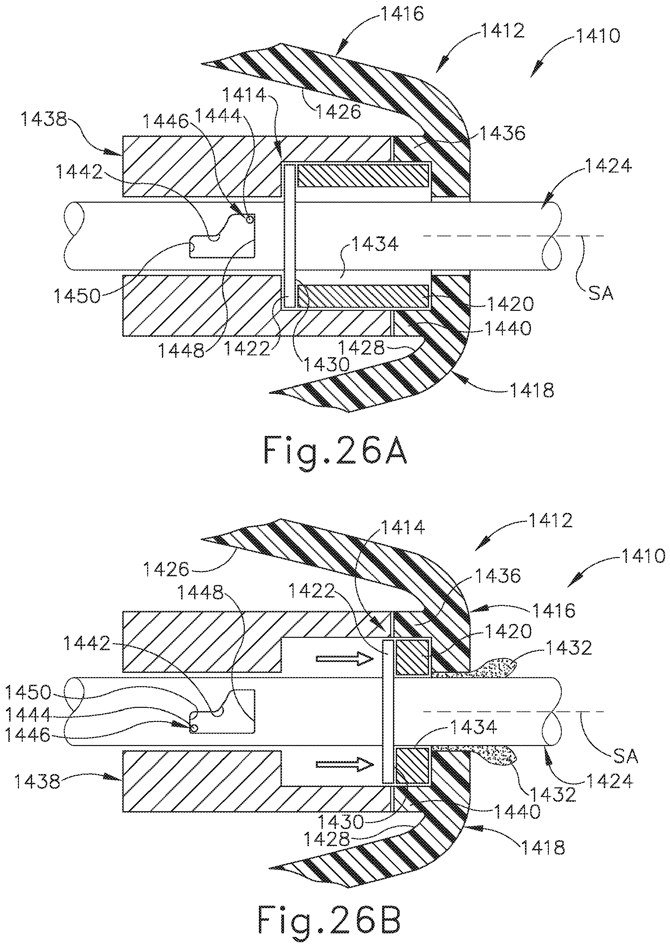

FIGS. 26A-26B show cross-sectional views of a sixth exemplary alternative shaft assembly (1410) that includes a tenth exemplary alternative nozzle (1412) and an eleventh exemplary fluid blocker (1414). Nozzle (1412) may include at least one housing (shown as upper and lower distal nozzle housings (1416, 1418)). As shown, upper and lower distal nozzle housings (1416, 1418) are identical and may be coupled together (e.g. snapped together) to form nozzle (1412). Upper and lower distal nozzle housings (1416, 1418) may be different if desired. As shown, fluid blocker (1414) includes a fluid seal (1420) and a fluid shield (1422), where fluid shield (1422) is coupled with a closure tube (1424) (similar to closure tube (62)). Fluid seal (1420) and fluid shield (1422) may be annular.

FIG. 26A shows a schematic cross-sectional view of shaft assembly (1410), where closure tube (1424) of shaft assembly (1410) is in a proximal position causing an end effector (not shown, but similar to end effector (16)) to be in an open configuration. As shown in FIG. 26A, fluid seal (1420) is in a stretched or unstretched state and seals against inner surfaces (1426, 1428) of upper and lower distal nozzle housings (1416, 1418) and outwardly facing surface (1430) of fluid shield (1422). Upper distal nozzle housing (1416) includes an inner projection (1436) that faces proximally that is configured to be in contact with a switch collar (1438). Similarly, lower distal nozzle housing (1418) includes an inner projection (1440) that faces proximally that is configured to be in contact with switch collar (1438). Closure tube (1424) includes a cutout (1442) for a shifter pin (1444) of a plate (1446). As shown in FIG. 24A, shifter pin (1444) is located adjacent a distal surface (1448) of cutout (1442) of closure tube (1424).

FIG. 26B shows a schematic cross-sectional view of shaft assembly (1410) of FIG. 26A, where closure tube (1424) of shaft assembly (1410) is in a distal position causing the end effector (not shown, but similar to end effector (16)) to be in a closed configuration. As shown in FIG. 26B, fluid seal (1420) is in a compressed state that seals against inner surfaces (1426, 1428) of upper and lower distal nozzle housings (1416, 1418) and outwardly facing surface (1430) of fluid shield (1422). In the compressed state, fluid seal (1420) causes fluid (1432) to be pushed distally out of an annular cavity (1434). As shown in FIG. 24A, shifter pin (1444) is located adjacent a proximal surface (1450) of cutout (1442) of closure tube (1424).

Fluid seal (1420) may be a compressible cylindraceous seal that is interposed between fluid shield (1422) and upper and lower distal nozzle housings (1416, 1418) of nozzle (1412). Fluid shield (1422) may be a disc-shaped component that is fixedly secured to closure tube (1424), which translates longitudinally relative to upper and lower distal nozzle housings (1416, 1418) of nozzle (1412) to provide closure of anvil (not shown, but similar to anvil (74)). While FIGS. 26A-26B show closure tube (1424) as being a solid, monolithic piece of material, closure tube (1424) is intended to be hollow. Additionally, FIGS. 26A-26B omit the components that are located within the hollow interior of closure tube (1424).

### L. Eleventh Exemplary Alternative Nozzle and Twelfth Exemplary Fluid Blocker

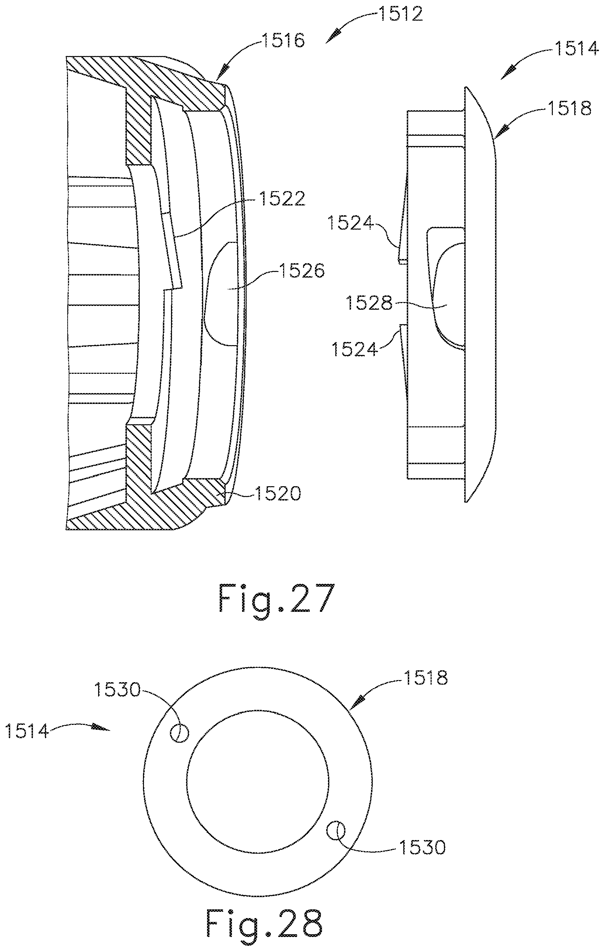

FIG. 27 shows a bottom view of an eleventh exemplary alternative nozzle (1512) and a twelfth exemplary fluid blocker (1514). Nozzle (1512) includes at least one housing (shown as upper distal nozzle housing (1516)). While not shown, nozzle (1512) may also include a lower distal nozzle housing (similar to upper distal nozzle housing (1516) and/or a proximal nozzle housing (similar to proximal nozzle housing (164)). For example, lower distal nozzle housing may be identical to upper distal nozzle housing (1516).

As shown in FIG. 27, fluid blocker (1514) includes a body (1518) that extends over a distal end (1520) of nozzle (1512). Body (1518) of fluid blocker (1514) rotatably engages with distal end (1520) of nozzle (1512). Upper distal nozzle housing (1516) includes a ramp (1522) that is configured to engage a locking ratchet (1524) of body (1518) of fluid blocker (1514) to prevent back rotation. Upper distal nozzle housing (1516) includes an alignment feature (1526) that is configured to engage a ramp (1528) of body (1518) of fluid blocker (1514). Alignment feature (1526) aligns body (1518) of fluid blocker (1514) to upper distal nozzle housing (1516) and rides up ramp (1528) to tighten body (1518) to upper distal nozzle housing (1516). Ramp (1528) of body (1518) of fluid blocker (1514) allows alignment feature (1526) of upper distal nozzle housing (1516) to pull body (1518) of fluid blocker (1514) tight against upper distal nozzle housing (1516) of nozzle (1512). Body (1518) of fluid blocker (1514) may be formed of a hard-polymeric material (e.g. plastic).

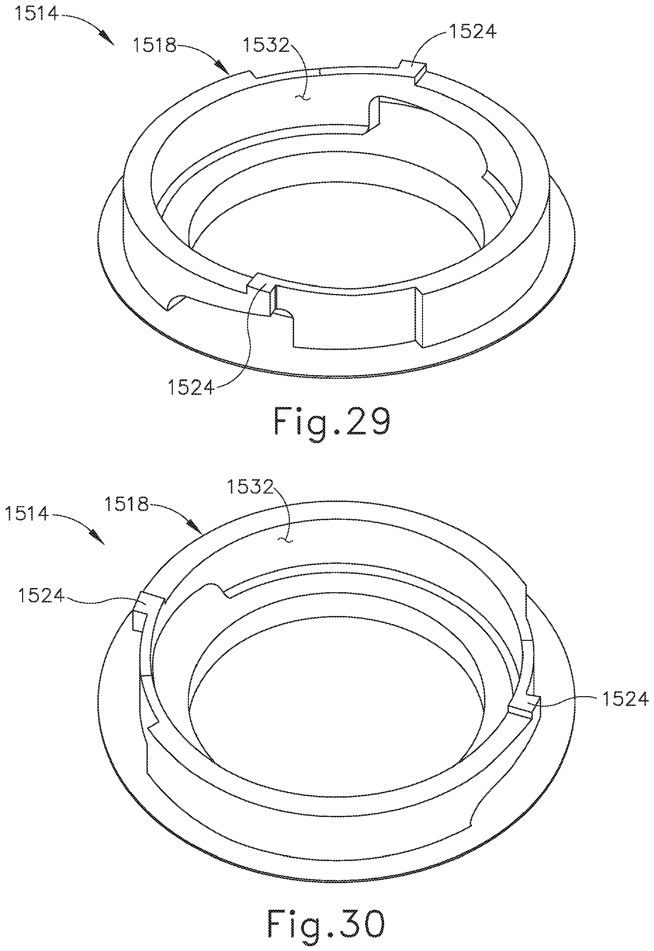

FIG. 28 shows a front view of fluid blocker of FIG. 27. As shown in FIG. 28, body (1518) of fluid blocker (1514) may also include spanner wrench locking apertures (1530) for assembly. FIGS. 29-30 show perspective views of fluid blocker (1514) of FIG. 27. Annular body (326) of fluid blocker (314, 514), described above with reference to FIGS. 6-11, may be inserted into body (1518) of fluid blocker (1514) and contact inner surface (1532) of body (1518).

Nozzle (1512) may provide many benefits. For example, fluid blocker (1514) protects internal components by preventing, or at least reducing, fluid within shaft assembly (910) arising from ingress between a closure tube (not shown, but similar to closure tube (62)) and distal nozzle opening of nozzle (1512). Additionally, nozzle (1512) may include the same internal shaft components as shaft assembly (14) and utilize the same assembly process as shaft assembly (14) with minimal added components (e.g. fluid blocker (1514)). Additionally, since the internal geometry of nozzle (1512) accommodates fluid blocker (314), this geometry change may stabilize and/or center the closure tube (not shown) within nozzle (1512). This may result in more consistent shifting and reduced interference of the switch collar with nozzle (1512) while the closure tube is under load. The interface gap between the closure tube and upper distal nozzle housing (1516) and lower distal nozzle housing (not shown) is reduced relative to nozzle (60). Body (1518) helps to shed fluid from distal end (1520) of nozzle (1512).

### IV. Miscellaneous

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those of ordinary skill in the art in view of the teachings herein.

Versions of the devices described above may have application in conventional medical treatments and procedures conducted by a medical professional, as well as application in robotic-assisted medical treatments and procedures. By way of example only, various teachings herein may be readily incorporated into a robotic surgical system such as the DAVINCI^{™} system by Intuitive Surgical, Inc., of Sunnyvale, California. Similarly, those of ordinary skill in the art will recognize that various teachings herein may be readily combined with various teachings of any of the following: U.S. Pat. No. 5,792,135, entitled "Articulated Surgical Instrument For Performing Minimally Invasive Surgery With Enhanced Dexterity and Sensitivity," issued Aug. 11, 1998, the disclosure of which is referred to herein; U.S. Pat. No. 5,817,084, entitled "Remote Center Positioning Device with Flexible Drive," issued Oct. 6, 1998, the disclosure of which is referred to herein; U.S. Pat. No. 5,878,193, entitled "Automated Endoscope System for Optimal Positioning," issued March 2, 1999, the disclosure of which is referred to herein; U.S. Pat. No. 6,231,565, entitled "Robotic Arm DLUS for Performing Surgical Tasks," issued May 15, 2001, the disclosure of which is referred to herein; U.S. Pat. No. 6,783,524, entitled "Robotic Surgical Tool with Ultrasound Cauterizing and Cutting Instrument," issued Aug. 31, 2004, the disclosure of which is referred toherein; U.S. Pat. No. 6,364,888, entitled "Alignment of Master and Slave in a Minimally Invasive Surgical Apparatus," issued April 2, 2002, the disclosure of which is referred to herein; U.S. Pat. No. 7,524,320, entitled "Mechanical Actuator Interface System for Robotic Surgical Tools," issued April 28, 2009, the disclosure of which is referred to herein; U.S. Pat. No. 7,691,098, entitled "Platform Link Wrist Mechanism," issued April 6, 2010, the disclosure of which is referred toherein; U.S. Pat. No. 7,806,891, entitled "Repositioning and Reorientation of Master/Slave Relationship in Minimally Invasive Telesurgery," issued Oct. 5, 2010, the disclosure of which is referred toherein; U.S. Pat. No. 8,844,789, entitled "Automated End Effector Component Reloading System for Use with a Robotic System," issued Sept. 30, 2014, the disclosure of which is referred to herein; U.S. Pat. No. 8,820,605, entitled "Robotically-Controlled Surgical Instruments," issued Sept. 2, 2014, the disclosure of which is referred to herein; U.S. Pat. No. 8,616,431, entitled "Shiftable Drive Interface for Robotically-Controlled Surgical Tool," issued Dec. 31, 2013, the disclosure of which is referred to herein; U.S. Pat. No. 8,573,461, entitled "Surgical Stapling Instruments with Cam-Driven Staple Deployment Arrangements," issued Nov. 5, 2013, the disclosure of which is referred to herein; U.S. Pat. No. 8,602,288, entitled "Robotically-Controlled Motorized Surgical End Effector System with Rotary Actuated Closure Systems Having Variable Actuation Speeds," issued Dec. 10, 2013, the disclosure of which is referred to herein; U.S. Pat. No. 9,301,759, entitled "Robotically-Controlled Surgical Instrument with Selectively Articulatable End Effector," issued April 5, 2016, the disclosure of which is referred to herein; U.S. Pat. No. 8,783,541, entitled "Robotically-Controlled Surgical End Effector System," issued July 22, 2014, the disclosure of which is referred to herein; U.S. Pat. No. 8,479,969, entitled "Drive Interface for Operably Coupling a Manipulatable Surgical Tool to a Robot," issued July 9, 2013; U.S. Pat. Pub. No. 8,800,838, entitled "Robotically-Controlled Cable-Based Surgical End Effectors," issued Aug. 12, 2014, the disclosure of which is referred to herein; and/or U.S. Pat. No. 8,573,465, entitled "Robotically-Controlled Surgical End Effector System with Rotary Actuated Closure Systems," issued Nov. 5, 2013, the disclosure of which is referred to herein.

Versions of the devices described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by a user immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A surgical instrument (10), comprising:
(a) a body assembly (12) that includes at least one electrical connection;
(b) an end effector (16) operable to treat tissue including first and second opposing jaws ; and
(c) a shaft assembly (310) extending between the body assembly (12) and the end effector (16) along a shaft axis (SA), wherein the shaft assembly (310) comprises:
(A) a nozzle (312) that includes at least one housing, wherein the at least one housing includes a recess (371, 403),
(B) a closure tube (322) configured to rotate relative to the body assembly (12) about the shaft axis (SA), and
(C) a fluid blocker (514) disposed within the recess (371, 403) of the nozzle, wherein the fluid blocker (514) is configured to contact the closure tube (322) to prevent fluid from entering the nozzle (312) and reaching the at least one electrical connection disposed within the body assembly (12), wherein the fluid blocker (514) includes an annular member (514) that entirely surrounds the closure tube (322) and the at least one housing includes first (316) and second (318) proximal housings that collectively form the recess (371, 403) configured to receive the annular member (514), and
**characterized in that** the annular member (514) includes first (516) and second (518) portions that are completely separable from one another, wherein the first proximal housing (316) of the nozzle (312) captures the first portion of the annular member (516) and the second proximal housing (318) of the nozzle (312) captures the second portion of the annular member (518).

2. The surgical instrument (10) of claim 1, wherein the annular member (514) is configured to wipe an outer perimeter of the closure tube (322) as the closure tube (322) moves longitudinally along the shaft axis (SA).

3. The surgical instrument (10) of claim 1, wherein the second proximal housing (318) includes first (400) and second retention (402) features that are configured to retain the annular member (514) within the recess (403).

4. The surgical instrument (10) of claim 3, wherein the first retention feature (400) includes first and second opposing holders (404a, 404b) configured to receive a first portion of the annular member (514) therebetween, wherein the second retention (402) feature includes third and fourth opposing holders (404c, 404d) configured to receive a second portion of the annular member (514) therebetween.

5. The surgical instrument (10) of claim 1, wherein the first proximal housing includes (316) first and second retention features (368, 370) that are configured to retain the annular member (514) within the recess (371).

6. The surgical instrument (10) of claim 5, wherein the first retention feature (368) includes first and second opposing holders (374a, 374b) configured to receive a first portion of the annular member (514) therebetween, wherein the second retention feature (370) includes third and fourth opposing holders (374c, 374d) configured to receive a second portion of the annular member (514) therebetween.

7. The surgical instrument (10) of claim 1, wherein the annular member (514) is configured to provide a seal between the closure tube (322) and the nozzle (312) to prevent the fluid from reaching the at least one electrical connection disposed in the body assembly (12).

8. The surgical instrument (10) of claim 1, wherein the annular member (514) is configured to wick the fluid to prevent the fluid from reaching the at least one electrical connection disposed in the body assembly (12).

9. The surgical instrument (10) of claim 1, wherein the annular member (514) includes a biocompatible fluid absorbing ring that is configured to absorb the fluid to prevent the fluid from reaching the at least one electrical connection disposed in the body assembly (12).

10. The surgical instrument (10) of claim 1, wherein the nozzle (312) includes an outer covering on the nozzle (312) to prevent the fluid from reaching the at least one electrical connection disposed within the body assembly (12), wherein the fluid blocker (514) is configured to be compressed between the nozzle (312) and the outer covering.

11. The surgical instrument (10) of claim 1, wherein the first jaw includes an elongate channel (70) that is configured to receive a staple cartridge (72), wherein the second jaw includes an anvil (74) configured to pivot relative to channel between open and closed positions for clamping tissue between the anvil (74) and the staple cartridge (72).

## Patentansprüche

1. Chirurgisches Instrument (10), umfassend:
(a) eine Körperanordnung (12), die mindestens eine elektrische Verbindung aufweist;
(b) einen Endeffektor (16), der bedienbar ist, um Gewebe zu behandeln, und eine erste und eine zweite gegenüberliegende Backe aufweist; und
(c) eine Schaftanordnung (310), die sich zwischen der Körperanordnung (12) und dem Endeffektor (16) entlang einer Schaftachse (SA) erstreckt, wobei die Schaftanordnung (310) umfasst:
(A) eine Düse (312), die mindestens ein Gehäuse aufweist, wobei das mindestens eine Gehäuse eine Aussparung (371, 403) aufweist,
(B) ein Schließrohr (322), das dazu ausgelegt ist, bezogen auf die Körperanordnung (12) um die Schaftachse (SA) zu drehen, und
(C) einen Fluidblocker (514), der in der Aussparung (371, 403) der Düse angeordnet ist, wobei der Fluidblocker (514) dazu ausgelegt ist, mit dem Schließrohr (322) in Kontakt zu treten, um zu verhindern, dass Fluid in die Düse (312) gelangt und die mindestens eine elektrische Verbindung erreicht, die in der Körperanordnung (12) angeordnet ist, wobei der Fluidblocker (514) ein ringförmiges Element (514) aufweist, das das Schließrohr (322) vollständig umgibt und das mindestens eine Gehäuse ein erstes (316) und ein zweites (318) proximales Gehäuse aufweist, die zusammen die Aussparung (371, 403) bilden, die dazu ausgelegt ist, das ringförmige Element (514) aufzunehmen, und
**dadurch gekennzeichnet, dass** das ringförmige Element (514) einen ersten (516) und einen zweiten (518) Abschnitt aufweist, die vollständig voneinander trennbar sind, wobei das erste proximale Gehäuse (316) der Düse (312) den ersten Abschnitt des ringförmigen Elements (516) erfasst und das zweite proximale Gehäuse (318) der Düse (312) den zweiten Abschnitt des ringförmigen Elements (518) erfasst.

2. Chirurgisches Instrument (10) nach Anspruch 1, wobei das ringförmige Element (514) dazu ausgelegt ist, einen äußeren Umfang des Schließrohrs (322) zu säubern, wenn sich das Schließrohr (322) in Längsrichtung entlang der Schaftachse (SA) bewegt.

3. Chirurgisches Instrument (10) nach Anspruch 1, wobei das zweite proximale Gehäuse (318) eine erste (400) und eine zweite Haltefunktion (402) aufweist, die dazu ausgelegt sind, das ringförmige Element (514) in der Aussparung (403) zu halten.

4. Chirurgisches Instrument (10) nach Anspruch 3, wobei die erste Haltefunktion (400) eine erste und eine zweite gegenüberliegende Halterung (404a, 404b) aufweist, die dazu ausgelegt sind, einen ersten Abschnitt des ringförmigen Elements (514) dazwischen aufzunehmen, wobei die zweite Haltefunktion (402) eine dritte und eine vierte gegenüberliegende Halterung (404c, 404d) aufweist, die dazu ausgelegt sind, einen zweiten Abschnitt des ringförmigen Elements (514) dazwischen aufzunehmen.

5. Chirurgisches Instrument (10) nach Anspruch 1, wobei das erste proximale Gehäuse (316) eine erste und eine zweite Haltefunktion (368, 370) aufweist, die dazu ausgelegt sind, das ringförmige Element (514) in der Aussparung (371) zu halten.

6. Chirurgisches Instrument (10) nach Anspruch 5, wobei die erste Haltefunktion (368) eine erste und eine zweite gegenüberliegende Halterung (374a, 374b) aufweist, die dazu ausgelegt sind, einen ersten Abschnitt des ringförmigen Elements (514) dazwischen aufzunehmen, wobei die zweite Haltefunktion (370) eine dritte und eine vierte gegenüberliegende Halterung (374c, 374d) aufweist, die dazu ausgelegt sind, einen zweiten Abschnitt des ringförmigen Elements (514) dazwischen aufzunehmen.

7. Chirurgisches Instrument (10) nach Anspruch 1, wobei das ringförmige Element (514) dazu ausgelegt ist, eine Dichtung zwischen dem Schließrohr (322) und der Düse (312) bereitzustellen, um zu verhindern, dass das Fluid die mindestens eine elektrische Verbindung erreicht, die in der Körperanordnung (12) angeordnet ist.

8. Chirurgisches Instrument (10) nach Anspruch 1, wobei das ringförmige Element (514) dazu ausgelegt ist, das Fluid abzuleiten, um zu verhindern, dass das Fluid die mindestens eine elektrische Verbindung erreicht, die in der Körperanordnung (12) angeordnet ist.

9. Chirurgisches Instrument (10) nach Anspruch 1, wobei das ringförmige Element (514) einen biokompatibles Fluid absorbierenden Ring aufweist, der dazu ausgelegt ist, das Fluid zu absorbieren, um zu verhindern, dass das Fluid die mindestens eine elektrische Verbindung erreicht, die in der Körperanordnung (12) angeordnet ist.

10. Chirurgisches Instrument (10) nach Anspruch 1, wobei die Düse (312) eine äußere Abdeckung an der Düse (312) aufweist, um zu verhindern, dass das Fluid die mindestens eine elektrische Verbindung erreicht, die in der Körperanordnung (12) angeordnet ist, wobei der Fluidblocker (514) dazu ausgelegt ist, zwischen der Düse (312) und der äußeren Abdeckung zusammengedrückt zu werden.

11. Chirurgisches Instrument (10), wobei die erste Backe einen länglichen Kanal (70) aufweist, der dazu ausgelegt ist, ein Klammermagazin (72) aufzunehmen, wobei die zweite Backe einen Amboss (74) aufweist, der dazu ausgelegt ist, bezogen auf den Kanal zwischen einer offenen und einer geschlossenen Position zu schwenken, um Gewebe zwischen dem Amboss (74) und dem Klammermagazin (72) einzuklemmen.

## Revendications

1. Instrument chirurgical (10) comprenant :
(a) un ensemble de corps (12) qui comprend au moins une connexion électrique ;
(b) un effecteur d'extrémité (16) utilisable pour traiter les tissus, comprenant des première et seconde mâchoires opposées ; et
(c) un ensemble d'arbre (310) s'étendant entre l'ensemble de corps (12) et l'effecteur d'extrémité (16) le long d'un axe d'arbre (SA), l'ensemble d'arbre (310) comprenant :
(A) une buse (312) qui comprend au moins un logement, l'au moins un logement comprenant un renfoncement (371, 403),
(B) un tube de fermeture (322) configuré pour tourner par rapport à l'ensemble de corps (12) autour de l'axe de l'arbre (SA), et
(C) un bloqueur de fluide (514) disposé dans le renfoncement (371, 403) de la buse, le bloqueur de fluide (514) étant configuré pour entrer en contact avec le tube de fermeture (322) afin d'empêcher le fluide de pénétrer dans la buse (312) et d'atteindre l'au moins une connexion électrique disposée à l'intérieur de l'ensemble de corps (12), le bloqueur de fluide (514) comprenant un élément annulaire (514) qui entoure entièrement le tube de fermeture (322) et l'au moins un logement comprenant des premier (316) et second (318) logements proximaux qui forment collectivement le renfoncement (371, 403) configuré pour recevoir l'élément annulaire (514), et
**caractérisé en ce que** l'élément annulaire (514) comprend des première (516) et seconde (518) parties qui sont complètement séparables l'une de l'autre, le premier logement proximal (316) de la buse (312) capturant la première partie de l'élément annulaire (516) et le second logement proximal (318) de la buse (312) capturant la seconde partie de l'élément annulaire (518).

2. Instrument chirurgical (10) selon la revendication 1, l'élément annulaire (514) étant configuré pour essuyer un périmètre extérieur du tube de fermeture (322) lorsque le tube de fermeture (322) se déplace longitudinalement le long de l'axe de l'arbre (SA).

3. Instrument chirurgical (10) selon la revendication 1, le second logement proximal (318) comprenant des première (400) et seconde caractéristiques de retenue (402) qui sont configurées pour retenir l'élément annulaire (514) dans le renfoncement (403).

4. Instrument chirurgical (10) selon la revendication 3, la première caractéristique de retenue (400) comprenant des premier et deuxième supports opposés (404a, 404b) configurés pour recevoir une première partie de l'élément annulaire (514) entre eux, la deuxième caractéristique de retenue (402) comprenant des troisième et quatrième supports opposés (404c, 404d) configurés pour recevoir une deuxième partie de l'élément annulaire (514) entre eux.

5. Instrument chirurgical (10) selon la revendication 1, le premier logement proximal comprenant (316) des première et deuxième caractéristiques de retenue (368, 370) qui sont configurées pour retenir l'élément annulaire (514) dans le renfoncement (371).

6. Instrument chirurgical (10) selon la revendication 5, la première caractéristique de retenue (368) comprenant des premier et deuxième supports opposés (374a, 374b) configurés pour recevoir une première partie de l'élément annulaire (514) entre eux, la deuxième caractéristique de retenue (370) comprenant des troisième et quatrième supports opposés (374c, 374d) configurés pour recevoir une deuxième partie de l'élément annulaire (514) entre eux.

7. Instrument chirurgical (10) selon la revendication 1, l'élément annulaire (514) étant configuré pour fournir un joint entre le tube de fermeture (322) et la buse (312) pour empêcher le fluide d'atteindre l'au moins une connexion électrique disposée dans l'ensemble de corps (12).

8. Instrument chirurgical (10) selon la revendication 1, l'élément annulaire (514) étant configuré pour absorber le fluide afin d'empêcher le fluide d'atteindre l'au moins une connexion électrique disposée dans l'ensemble de corps (12).

9. Instrument chirurgical (10) selon la revendication 1, l'élément annulaire (514) comprenant un anneau d'absorption de fluide biocompatible qui est configuré pour absorber le fluide afin d'empêcher le fluide d'atteindre l'au moins une connexion électrique disposée dans l'ensemble de corps (12).

10. Instrument chirurgical (10) selon la revendication 1, la buse (312) comprenant un revêtement extérieur sur la buse (312) pour empêcher le fluide d'atteindre l'au moins une connexion électrique disposée dans l'ensemble de corps (12), le bloqueur de fluide (514) étant configuré pour être comprimé entre la buse (312) et le revêtement extérieur.

11. Instrument chirurgical (10) selon la revendication 1, la première mâchoire comprenant un canal allongé (70) qui est configuré pour recevoir une cartouche d'agrafes (72), la seconde mâchoire comprenant une enclume (74) configurée pour pivoter par rapport au canal entre des positions ouverte et fermée pour serrer le tissu entre l'enclume (74) et la cartouche d'agrafes (72) .
